# EUROPÄISCHE PATENTSCHRIFT

(11) **EP 1 474 518 B1**
(45) Veröffentlichungstag und Bekanntmachung des Hinweises auf die Patenterteilung: **16.04.2008**
(21) Anmeldenummer: 03737327.1
(22) Anmeldetag: 07.02.2003
(51) Int. Cl.: C12N 15/67, C12N 15/70

(54) **VERFAHREN ZUR VORHERSAGE DER EXPRESSIONSEFFIZIENZ IN ZELLFREIEN EXPRESSIONSSYSTEMEN**
METHOD FOR PREDICTING THE EXPRESSION EFFICIENCY IN CELL-FREE EXPRESSION SYSTEMS
PROCEDE POUR PREVOIR L'EFFICACITE D'EXPRESSION DANS DES SYSTEMES D'EXPRESSION EXEMPTS DE CELLULE

(30) Priorität: 07.02.2002 DE 10205091
(43) Veröffentlichungstag der Anmeldung: 10.11.2004
(73) Patentinhaber: Biomax Informatics AG, 82152 Martinsried (DE)
(72) Erfinder: VOGES, Dieter, D-80639 München (DE); BUCHBERGER, Bernd, 82380 Peissenberg (DE); WIZEMANN, Sabine, D-83673 Bichl (DE); WATZELE, Manfred, D-82362 Weilheim (DE); NEMETZ, Cordula, 82515 Wolfratshausen (DE)
(74) Vertreter: Huenges, Martin
(86) Internationale Anmeldenummer: PCT/EP2003/001251
(87) Internationale Veröffentlichungsnummer: WO 2003/066864

(56) Entgegenhaltungen:
- WO-A-89/00604
- PEDERSEN-LANE JOAN ET AL: "High-level expression of human thymidylate synthase." PROTEIN EXPRESSION AND PURIFICATION, Bd. 10, Nr. 2, 1997, Seiten 256-262, XP002240373 ISSN: 1046-5928
- ROBINSON M ET AL: "CODON USAGE CAN AFFECT EFFICIENCY OF TRANSLATION OF GENES IN ESCHERICHIA COLI" NUCLEIC ACIDS RESEARCH, OXFORD UNIVERSITY PRESS, SURREY, GB, Bd. 12, Nr. 17, 11. September 1984 (1984-09-11), Seiten 6663-6671, XP009006930 ISSN: 0305-1048
- STENSTROM C M ET AL: "Cooperative effects by the initiation codon and its flanking regions on translation initiation" GENE 08 AUG 2001 NETHERLANDS, Bd. 273, Nr. 2, 8. August 2001 (2001-08-08), Seiten 259-265, XP004302709 ISSN: 0378-1119
- BONEKAMP F ET AL: "Codon-defined ribosomal pausing in Escherichia coli detected by using the pyrE attenuator to probe the coupling between transcription and translation." NUCLEIC ACIDS RESEARCH. ENGLAND 11 JUN 1985, Bd. 13, Nr. 11, 11. Juni 1985 (1985-06-11), Seiten 4113-4123, XP009010095 ISSN: 0305-1048
- LOOMAN A C ET AL: "INFLUENCE OF THE CODON FOLLOWING THE AUG INITIATION CODON ON THE EXPRESSION OF A MODIFIED LACZ GENE IN ESCHERICHIA COLI" EMBO JOURNAL, OXFORD UNIVERSITY PRESS, SURREY, GB, Bd. 6, Nr. 8, 1987, Seiten 2489-2492, XP002946308 ISSN: 0261-4189
- SPANJAARD R A ET AL: "EXPRESSION OF THE RAT INTERFERON-ALPHA-1 GENE IN ESCHERICHIA-COLI CONTROLLED BY THE SECONDARY STRUCTURE OF THE TRANSLATION-INITIATION REGION" GENE, ELSEVIER BIOMEDICAL PRESS. AMSTERDAM, NL, Bd. 80, Nr. 2, 1989, Seiten 345-351, XP002182303 ISSN: 0378-1119

## Beschreibung

Die Erfindung betrifft ein Verfahren zur Analyse und Optimierung der Expressionseffizienz bei der Herstellung eines Proteins in Expressionssystemen sowie ein Verfahren zur Herstellung von Proteinen mit derartigen Expressionssystemen.

Zur Herstellung von Proteinen stehen eine Vielzahl von Expressionssystemen zur Verfügung. Dabei unterscheidet man zwischen Herstellungsverfahren, die lebende Zellen wie *E. coli,* Hefe, Säugerzellkulturen und Insektenzellen verwenden, d.h. auf zellulären bzw. in vivo- Expressionssystemen basieren, und Herstellungsverfahren, die subzelluläre Fraktionen aus geeigneten Organismen wie einem *E. coli*-Lysat, einem Weizenkeim-Lysat oder einem Retikulozyten-Lysat verwenden, d.h. auf zellfreien bzw. in vitro-Expressionssystemen basieren.

Die vorstehend genannten Verfahren weisen jeweils spezifische Vor- und Nachteile auf. So sind zelluläre Expressionssysteme weit verbreitet, können leicht in größere Maßstäbe skaliert werden und ermöglichen z.T. die Herstellung sekundärmodifizierter Produkte. Nachteile sind dagegen die Notwendigkeit einer entsprechenden Infrastruktur, eine häufig aufwändige Optimierung und das erforderliche Spezialwissen.

Zellfreie Expressionssysteme sind erst in jüngerer Vergangenheit soweit optimiert worden, dass sie in Bezug auf Produktivität und Skalierbarkeit mit zellulären Systemen konkurrieren können. Vorteile dieser Methodik sind die Einfachheit und Flexibilität der Handhabung, die einfache Markierung von Proteinen, die Expression von Zell-toxischen Proteinen und die Möglichkeit der parallelen Expression von beispielsweise Hunderten von Proteinen. Aufgrund der einfachen Wachstumsbedingungen und der gut etablierten molekularbiologischen und genetischen Methodik werden prokaryontische Systeme, speziell *E*. *coli,* bevorzugt eingesetzt. Als Folge der Aktivitäten auf dem Gebiet der Genom-Sequenzierung steigt der Bedarf nach einfachen, robusten und effizienten Proteinexpressionsverfahren weiter an.

Ein Schwachpunkt bei der Herstellung eines Proteins sowohl in zellulären als auch in zellfreien Expressionssystemen ist die geringe Vorhersagbarkeit der Expressionsausbeute. Dies gilt insbesondere dann, wenn Gene eines Organismus, z.B. Mensch, in einem heterologen System, z.B. *E. coli,* zur Expression gebracht werden sollen. Ursachen hierfür sind insbesondere die Spezies-spezifischen Eigenarten der Verwendung des genetischen Kodes, besondere Signalsequenzen und die oftmals vielfältigen Regulationsmechanismen. Häufig verfährt man nach dem Prinzip Versuch-und-Irrtum, so dass ein hoher zeitlicher und finanzieller Aufwand damit verbunden ist das gewünschte Ergebnis zu erzielen.

Die Herstellung von Peptiden und Proteinen in zellulären oder zellfreien prokaryontischen Expressionssystemen ist ausführlich im Stand der Technik beschrieben. Derartige Systeme verwenden DNA-Matrizen wie Plasmide oder mittels PCR generierte lineare Matrizen und häufig eine Bakteriophagen RNA-Polymerase. Bei der zellulären Expression werden Substrate, Cofaktoren und Hilfsenzyme von der Zelle bereitgestellt.

Dagegen muss bei der zellfreien Proteinsynthese ein Teil der Substrate wie Nukleotide und Aminosäuren dem Lysat zugeführt werden. Beispielsweise enthält ein Kit für die in vitro-Expression einen speziell präparierten *E*. *coli*-Extrakt, einen Mix aller notwendigen Substrate und ein sekundäres Energiesubstrat wie z.B. Creatinphosphat, Phosphoenolpyruvat oder Acetylphosphat. Die für das herzustellende Protein kodierende Sequenz liegt als Expressionskonstrukt vor, d.h. sie ist von den für die Expression nötigen regulatorischen Elementen in einem optimierten Abstand umgeben. Ein Expressionskonstrukt einer in *E. coli* oder einem Lysat aus *E. coli*-Kulturen mit Hilfe der T7-Polymerase zu exprimierenden Sequenz umfasst folglich idealerweise eine T7-Promotor-Sequenz, eine prokaryontische Ribosomen-Bindestelle und eine T7-Terminatorsequenz in einem optimierten Abstand voneinander. In einem ersten Schritt wird die Protein-kodierende Sequenz in einen für das gewählte Expressionssystem geeigneten Expressionsvektor kloniert bzw. es wird ein lineares Expressionskonstrukt über PCR-Techniken hergestellt. Dann wird in dem prokaryontischen Expressionssystem mit Hilfe der beschriebenen Komponenten die DNA in mRNA transkribiert und anschließend die mRNA in das Protein translatiert. Im zellfreien System laufen sowohl Transkription als auch Translation gekoppelt in einem Reaktionsgefäß ab. Die Substrate, die zur Aufrechterhaltung der Reaktion erforderlich sind, können auch kontinuierlich beispielsweise durch eine semipermeable Membran zugeführt werden. Die Transkription der DNA in mRNA erfolgt für die Expressionskonstrukte unterschiedlicher Gene etwa mit gleicher Effizienz. Daher hängt die Expressionsmenge jedes Proteins überwiegend von der Effizienz der Translation durch die *E. coli*-Ribosomen ab.

Im Folgenden werden die Begriffe Wirt und Wirtsorganismus auch bei der Beschreibung der zellfreien Expression verwendet und bezeichnen den Organismus, aus dem das bei der in vitro-Expression eingesetzte Zell-Extrakt bzw. Zell-Lysat stammt.

Mit den bislang bekannten Techniken ist es nicht möglich, die Menge an Protein, die durch ein Expressionssystem, insbesondere ein prokaryontisches Expressionssystem, produziert wird, vorherzusagen. Dies liegt zum einen daran, dass nicht alle Faktoren, die die Expression beeinflussen, bekannt sind und zum anderen daran, dass der genaue quantitative Einfluss sowie die Synergieeffekte der bekannten Faktoren nicht abgeschätzt werden können.

Bislang bekannte Einflussgrößen für die Effizienz der Expression sind z.B. die Art des Promotors, die Art des Proteins, die Kodonnutzung sowie die Sekundärstruktur der mRNA. Ferner gibt es Hinweise, dass die Effizienz der Translation empfindlich von deren Initiation abhängt, insbesondere von der Sekundärstruktur im Initiationsbereich. Die Initiationsregion liegt vor dem Translationsstartkodon und beinhaltet unter anderem die Shine-Dalgarno-Region. Möglicherweise sind auch die ersten beiden Kodons des Gens Teil der Initiationsregion.

Ein weiterer bekannter Einflussfaktor ist der so genannte Kodonadaptionsindex. Er gibt an, inwieweit eine Nukleotidsequenz an die Kodons gut exprimierter Proteine des Wirtsorganismus, aus dem beispielsweise der Translationsapparat für die in vitro-Synthese stammt, angepasst ist. Die Degeneration des genetischen Codes erlaubt den Austausch "ungünstiger" Kodons gegen "günstige" und so die Optimierung der Kodons für eine effiziente Synthese. Der Nachteil dieses Verfahrens liegt in dem großen experimentellen Aufwand, da das zu exprimierende Gen aus einer Vielzahl synthetisch hergestellter Nukleotidstücke zusammengesetzt werden muss.

Obwohl eine Vielzahl von Proteinen mittlerweile erfolgreich unter Verwendung von prokaryontischen Expressionssystemen exprimiert werden können, beträgt die Erfolgsrate bei Verwendung solcher Systeme gegenwärtig nur etwa 50%. In manchen Fällen kann die Proteinexpression durch die Verwendung von verschiedenen Expressionsvektoren, durch Einführung von N- oder C-terminalen Sequenz-Tags oder durch Substitution, Deletion oder Insertion einzelner oder mehrerer Nukleotide innerhalb der kodierenden Sequenz optimiert werden (Nemetz C., Watzele M., Wizemann S., Buchberger B., Metzler T., Zaiss K., Fernholz E., Mutter W.; Optimization of the translation initial region of procaryotic expression vectors for high level in vitro-protein synthesis; 18th Int. Congr. Of Biochem. and Mol. Biol. 2000). Eine derartige Versuch-und-Irrtum-Optimierung kann jedoch auch zu einer verminderten Expression oder dem teilweisen oder vollständigen Verlust der Funktion führen. Ferner sind die Produktausbeuten sehr schwierig vorherzusagen, da die zellfreie Transkription und Translation wie vorstehend beschrieben durch zahlreiche Faktoren und insbesondere durch das verwendete Expressionskonstrukt beeinflusst wird.

Looman A.C. et al., "Influence of the codon following the AUG initiation codon on the expression of a modified LacZ gene in Escherichia coli", EMBO Journal, Oxford University Press, Bd. 6, Nr. 8, 1987, Seiten 2489-2492, beschreibt den Einfluss des auf den Startkodon folgenden, zweiten Kodons auf die Expressionsausbeute. Die Expression von 64 am zweiten Kodon variierten Genen wurde durch Immunopräzipitation der Plasmid-kodierten Fusionsproteine gemessen. Dabei wurde gezeigt, dass die Effizienz der Genexpression um einen Faktor von mindestens 15 in Abhängigkeit der Sequenz des auf den Startkodon folgenden Kodons variiert. Ferner ist in qualitativer Weise der Effekt der Sekundärstruktur auf die Expressionseffizienz beschrieben.

Basierend auf den vorstehend beschriebenen Kenntnissen ist es somit bislang nicht möglich gewesen, die Expressionseffizienz anhand der das herzustellende Protein kodierenden Sequenz vorauszusagen.

Wünschenswert wäre somit ein Verfahren, mit dem die Erfolgsrate der Expression bei der Herstellung eines Proteins in Expressionssystemen vorhergesagt und gegebenenfalls Vorschläge für die Verbesserung der Expressionseffizienz bereits vor Durchführung des Experiments gemacht werden können.

Aufgabe der vorliegenden Erfindung ist es somit, ein Verfahren bereit zu stellen, mit dem die Expressionseffizienz bei der Herstellung eines Proteins in einem Expressionssystem, vorzugsweise in einem prokaryontischen Expressionssystem, besonders bevorzugt in einem zellfreien Expressionssystem anhand der gegebenen kodierenden DNA-Sequenz vorausgesagt werden kann. Eine weitere Aufgabe ist es, ein Verfahren bereit zu stellen, mit dem anhand der vorhergesagten Expressionseffizienz die Auswahl des Expressionskonstrukts optimiert werden kann. Weitere Aufgaben ergeben sich aus der folgenden Beschreibung.

Zur Lösung dieser und weiterer Aufgaben dienen die Merkmale der unabhängigen Schutzansprüche.

Vorteilhafte Ausgestaltungen sind in den jeweiligen Unteransprüchen definiert.

Es ist jetzt erstmals gelungen, ein Verfahren zur Verfügung zu stellen, mit dem die Expressionseffizienz bzw. Ausbeute bei der Herstellung eines Proteins in Expressionssystemen, vorzugsweise in prokaryontischen Expressionssystemen anhand einer vorgegebenen, das herzustellende Protein kodierenden Nukleotidsequenz mit hoher Genauigkeit vorhergesagt werden kann. Basierend auf dem erfindungsgemäßen Verfahren kann ferner ein optimiertes Expressionskonstrukt für die Proteinsynthese bereitgestellt werden. Dabei können auch benutzergewählte Nebenbedingungen berücksichtigt werden.

Unter einer hohen Genauigkeit wird im Rahmen der vorliegenden Erfindung eine Genauigkeit von mindestens 50%, vorzugsweise von mindestens 60%, mehr bevorzugt von mindestens 75%, besonders bevorzugt von mindestens 85% und am meisten bevorzugt von mindestens 95% verstanden. Als Genauigkeit der Vorhersage wird die Anzahl der korrekt vorhergesagten Expressionsausbeuten dividiert durch die Anzahl der vorhergesagten Expressionsausbeuten bezeichnet. Die Expressionsmenge bzw. Ausbeute für eine Sequenz gilt im Rahmen der vorliegenden Erfindung als korrekt vorhergesagt, falls die tatsächlich, d.h. experimentell beobachtete Expressionsmenge dieser Sequenz über bzw. unter einem bestimmten Schwellenwert liegt und die vorhergesagte Expressionsmenge derselben Sequenz ebenfalls über bzw. unter diesem Schwellenwert liegt.

Ferner kann ein Maß für die Vorhersagegenauigkeit angegeben werden, indem die Abweichung zwischen vorhergesagter und tatsächlicher Expressionsausbeute analysiert wird. So gilt die Expressionseffizienz bzw. die Ausbeute im Rahmen der vorliegenden Erfindung als richtig vorhergesagt, wenn die Abweichung von berechneter zu tatsächlicher Ausbeute höchstens 0,4 REE, vorzugsweise höchstens 0,35 REE und besonders bevorzugt höchstens 0,3 REE beträgt, wobei 1 REE für eine Referenzexpressionseinheit steht und der Expressionsmenge eines gut exprimierten Proteins in dem betrachteten Translationssystem entspricht. Hier wird als 1 REE die Expressionsmenge des grünen Fluoreszenzproteins (GFP) angenommen.

Ausgehend von der vorgegebenen kodierenden Sequenz werden zunächst ein oder mehrere, vorzugsweise mindestens 50, besonders bevorzugt mindestens 100 und am meisten bevorzugt mindestens 1000 Expressionskonstrukte bzw. Expressionsvektoren generiert. Unter der Generierung bzw. Erzeugung eines Expressionskonstrukts wird im Rahmen der vorliegenden Erfindung die Bereitstellung eines Konstrukts als mögliches Edukt für die Proteinsynthese verstanden. Das Expressionskonstrukt entspricht dabei der für das Protein kodierenden mRNA. Die Generierung erfolgt dabei üblicherweise nicht synthetisch, sondern vielmehr theoretisch im Rahmen der Syntheseplanung.

Vorzugsweise werden bei der Generierung vor und hinter den kodierenden Bereich Sequenzabschnitte mit regulatorischen Elementen wie beispielsweise einer Promotorsequenz, einer Ribosomenbindungsstelle und/oder einer Transkriptionsterminations-Sequenz gesetzt, die mit dem zu verwendenden Expressionssystem, beispielsweise einem auf *E. coli* und T7 RNA-Polymerase basierenden Expressionssystem, kompatibel sind.

Um die Erfolgschancen bei der Expression zu erhöhen, können bei der Generierung Mutationen im kodierenden Bereich sowie im regulatorischen Bereich vorgenommen werden. Die Mutationen werden beispielsweise so ausgewählt, dass sie zu identischen Aminosäuren, konservativen Aminosäureaustauschen, Aminosäure-Deletionen und/oder Aminosäure-Insertionen im Translationsprodukt führen. Falls Mutationen in der regulatorischen Sequenz vor dem Startkodon vorgenommen werden, muss auf die Funktionsfähigkeit dieser Elemente in Abhängigkeit von der Sequenz und von den Abständen zum Startkodon geachtet werden.

Wesentliche regulatorische Elemente sind ein Promotor für die Transkription, die Ribosomenbindungsstelle und mögliche Translations-steigernde Sequenzen. Ein bevorzugter Promotor für die prokaryontische Expression ist beispielweise der Promotor des Phagen T7, der eine hohe Transkriptionsrate gewährleistet. Die T7 Promotorsequenz wurde in Moffat, B.A. & Studier, F.W. (1986) J. Mol. Biol. 189, 113 beschrieben. Für die optimale Translationsrate wurden beispielsweise für den Prokaryonten *E.coli* eine optimale Ribosomenbindungsstelle (Shine, J & Dalgarno, L. (1974) Proc. Natl. Acad. Sci. USA 71, 1342) und ein optimaler Abstand dieser Bindungsstelle zum Start ATG (Chen, H. et al (1994) Nucl. Ac. Res. 22, 4953) gezeigt. Als zusätzliches Translations-steigerndes Element für *E. coli* wurde von Olins P.O. et al (1988) Gene 73, 227 eine regulatorische Region T7 Gen 10 identifiziert, die bei prokaryontischen Expressionskonstrukten häufig eingesetzt wird. Zwischen diesen Elementen und teilweise auch innerhalb dieser Elemente können ebenfalls Mutationen durchgeführt werden, um optimierte Expressionskonstrukte herzustellen.

Anschließend werden für jedes dieser Expressionskonstrukte mindestens ein, vorzugsweise mehrere Attributwerte bestimmt. Unter Attributwerten werden im Rahmen der vorliegenden Erfindung Eigenschaften bzw. Merkmale des Expressionskonstrukts verstanden, die die Effizienz der Expression eines Proteins, insbesondere in vitro, beeinflussen. Die Identifizierung dieser für die Expressionsmenge wichtigen Faktoren kann unter anderem durch statistische Routinen erfolgen. Beispiele für Attributwerte sind der G/C-Gehalt der kodierenden Sequenz, Kodonadaptionsindizes und Basenpaarungswahrscheinlichkeiten für jedes Kodon bzw. jede Base der Sequenz.

Durch wechselseitige Verknüpfung dieser Attributwerte wird die zu erwartende Expressionsmenge bzw. -ausbeute berechnet und gegebenenfalls eine Reihenfolge der Expressionskonstrukte entsprechend der zu erwartenden Expressionsmenge erstellt. Eine wechselseitige Verknüpfung der Attributwerte wird vorzugsweise aus der Analyse experimenteller Expressionsergebnisse, so genannter Trainingsdaten, abgeleitet. Dazu werden die Expressionsausbeuten für eine Vielzahl von Expressionskonstrukten experimentell bestimmt und anschließend die Abhängigkeit der Ausbeute von bestimmten Attributwerten der Expressionskonstrukte untersucht. Beispielsweise durch Regressionsverfahren lässt sich auf diese Weise eine wechselseitige Beziehung zur Berechnung der Expressionseffizienz bzw. -Ausbeute in Abhängigkeit von bestimmten Attributwerten ermitteln (W.W. Cooley, P.R. Lohnes, Multivariate data analysis, John Wiley, New York 1971, S. 49 ff.).

Gegenstand der vorliegenden Erfindung ist somit ein Verfahren zur Vorhersage der Expressionseffizienz bei der Herstellung eines Proteins in Expressionssystemen, vorzugsweise in prokaryontischen Expressionssystemen, das die folgenden Schritte umfasst:
A) Generierung von mindestens einem Expressionskonstrukt umfassend eine für das Protein kodierende Sequenz und flankierende Regulatorsequenzen;
B) Bestimmung von mindestens einem die Expressionseffizienz beeinflussenden Attributwert des generierten Expressionskonstrukts, wobei der Attributwert ausgewählt wird aus quantitativen Primärstrukturmerkmalen, qualitativen Primärstrukturmerkmalen und quantitativen Sekundärstrukturmerkmalen; und
C) Berechnung der Expressionseffizienz für das generierte Expressionskonstrukt, indem die Expressionseffizienz über einen Vorhersage-Algorithmus mit dem mindestens einen in Schritt B) bestimmten Attributwert verknüpft wird, wobei der Vorhersage-Algorithmus zur Berechnung der Expressionseffizienz aus der Abhängigkeit experimentell ermittelter Expressionsausbeuten von Expressionskonstrukten in dem Expressionssystem von den in Schritt B) bestimmten Attributwerten der Expressionskonstrukte durch multiple Regression, mittels eines neuronalen Netzes, mittels eines Bayesschen Netzes, und/oder mittels eines Computer-lernenden Verfahrens, das einen Entscheidungsbaum aus einem Satz von Fällen, die zu bekannten Klassen gehören aufbaut, abgeleitet worden ist.

Somit wird erfindungsgemäß ein Verfahren bereitgestellt, das eine hohe Genauigkeit für die Voraussage der Expressionsausbeute insbesondere in prokaryontischen Expressionssystemen erlaubt. Durch Variation bzw. Modifizierung der Konstrukte kann mit Hilfe des erfindungsgemäßen Verfahrens ein für das jeweilige Expressionssystem und das herzustellende Protein optimiertes Expressionskonstrukt bereitgestellt werden. Auf diese Weise kann die Expressionseffizienz in Expressionssystemen, insbesondere in prokaryontischen Expressionssystemen wesentlich verbessert werden. Neben der Expressionseffizienz können für eine vorgegebene kodierende Sequenz auch Informationen über das herzustellende Produkt wie beispielsweise dessen elektrophoretische Beweglichkeit, Löslichkeit, Abhängigkeit von Chaperonen und dergleichen bereitgestellt werden.

Das erfindungsgemäße Verfahren ist sowohl bei zellulärer Proteinexpression als auch bei zellfreier Proteinexpression zur Vorhersage der Expressionseffizienz geeignet. Als prokaryontische Expressionssysteme können somit zelluläre Expressionssysteme basierend auf prokaryontischen Zellen oder zellfreie Expressionssysteme basierend auf aus prokaryontischen Zellen gewonnenen Extrakten verwendet werden. Besonders bevorzugt wird als prokaryontische Zelle oder für die Herstellung eines Zell-Extrakts *E. coli* verwendet.

Die die Expressionseffizienz bestimmenden Attributwerte der kodierenden Sequenz werden insbesondere ausgewählt aus der Gruppe bestehend aus quantitativen Primärstruktur-Attributen, qualitativen Primärstruktur-Attributen und quantitativen Sekundärstruktur-Attributen.

Unter quantitativen Primärstruktur-Attributen werden im Rahmen der vorliegenden Erfindung Attribute verstanden, die durch die Häufigkeit des Vorkommens von Monomer-Bausteinen wie den Basen A, T, G und C in bestimmten Teilbereichen oder dem gesamten Bereich der Primärstruktur des Expressionskonstrukts bedingt sind. Ein quantitatives Primärstruktur-Attribut ist beispielsweise der G/C-Gehalt in Teilbereichen oder dem gesamten Bereich der kodierenden Sequenz.

Unter qualitativen Primärstruktur-Attributen werden im Rahmen der vorliegenden Erfindung Attribute verstanden, die mit der Art des Vorkommens von Monomer-Bausteinen wie den Basen A, T, G und C in bestimmten Teilbereichen oder dem gesamten Bereich der Primärstruktur des Expressionskonstrukts zusammenhängen. Ein Beispiel für ein qualitatives Primärstruktur-Attribut ist die erste Base des zweiten Kodons der kodierenden Sequenz und/oder die Basenfolge des zweiten Kodons.

Unter quantitativen Sekundärstruktur-Attributen werden im Rahmen der vorliegenden Erfindung Attribute verstanden, die durch die Sekundärstruktur des Expressionskonstrukts bzw. der transkribierten mRNA-Sequenz bedingt sind. Ein quantitatives Sekundärstruktur-Attribut ist beispielsweise die mRNA-Basenpaarungswahrscheinlichkeit für mindestens eine der Basen der kodierenden und der ihr vorausliegenden Sequenz, insbesondere der Sequenz, die im Bereich von 40 Basen upstream und downstream des Startkodons ATG liegt, besonders bevorzugt im Bereich von 100 Basen upstream und 60 Basen downstream des Startkodons, und am meisten bevorzugt innerhalb der ersten Basen der Protein-kodierenden Sequenz. Die Basenpaarungswahrscheinlichkeit gibt die Wahrscheinlichkeit für eine Basenpaarung innerhalb des Nukleinsäure-Stranges der mRNA an, wobei die Expressionseffizienz umso niedriger ist, je mehr derartige Basenpaarungen ausgebildet werden.

Wie bereits vorstehend erwähnt, können in Schritt A) des erfindungsgemäßen Verfahrens zur Optimierung der Expressionseffizienz Mutationen im kodierenden Bereich sowie im regulatorischen Bereich vorgenommen werden. Bei einer bevorzugten Ausführungsform des erfindungsgemäßen Verfahrens wird somit die Expressionseffizienz für kodierende Sequenzen bestimmt, die sich von der nativen, das herzustellende Protein kodierenden Sequenz durch mindestens einen Basenaustausch in der kodierenden Sequenz unterscheiden. Besonders bevorzugt sind Basenaustausche, die zu identischen Aminosäuren oder zu konservativen Aminosäureaustauschen im herzustellenden Protein führen. Als konservative Aminosäureaustausche werden im Rahmen der vorliegenden Erfindung solche bezeichnet, bei denen Aminosäuren durch solche mit ähnlichen Funktionalitäten, Ladungen, Polaritäten bzw. Hydrophobizitäten ersetzt werden, wie beispielsweise ein Austausch eines Glutaminrests durch einen Asparaginrest. Bei einer alternativen Ausführungsform des erfindungsgemäßen Verfahrens sind auch Aminosäureaustausche denkbar, bei denen eine Aminosäure durch eine beliebige andere Aminosäure substituiert wird. Anzahl bzw. Art der erlaubten Aminosäuresubstitutionen sind somit wesentliche Parameter dieser bevorzugten Ausführungsform der Generierung des Expressionskonstrukts in Schritt A) des erfindungsgemäßen Verfahrens.

Vorzugsweise befinden sich die Basenaustausche in den ersten Kodons, beispielsweise in den ersten 10 oder 20 Kodons, der kodierenden Sequenz. Ferner ist es bevorzugt, dass höchstens in sieben Kodons der kodierenden Sequenz Basenaustausche vorgenommen werden. Des Weiteren ist es bevorzugt, dass der G/C-Gehalt für jeden Kodon, in dem ein oder mehrere Basenaustausche vorgenommen werden, höchstens 0,7 beträgt. Ein weiteres bevorzugtes Merkmal bei der Einführung von Mutationen in den kodierenden Bereich ist, dass der Kodonadaptionsindex für jeden Kodon, in dem ein oder mehrere Basenaustausche vorgenommen werden, mindestens 0,02 beträgt. Der Kodonadaptionsindex gibt die Verwendung von Kodons in einem bestimmten Gen bezogen auf die allgemeine Verwendung dieser Kodons in exprimierten Genen eines Wirtsorganismus an.

Die Berechnung der Expressionseffizienz für jedes der Expressionskonstrukte durch wechselseitige Verknüpfung mit den bestimmten Attributwerten erfolgt vorzugsweise durch multiple Regression, beispielsweise durch lineare Regression der Abhängigkeit experimentell ermittelter Expressionsausbeuten von Attributwerten der dabei eingesetzten Expressionskonstrukte. Die Regressionsanalyse ist für das erfindungsgemäße Verfahren umso besser geeignet, je mehr experimentelle Daten für seine Bestimmung verwendet werden, d.h. je größer die Anzahl an Expressionskonstrukten ist, für die die Abhängigkeit der Expressionsausbeute von bestimmten Attributwerten experimentell ermittelt wird. Als Attributwerte bzw. unabhängige Variable bei der Regression werden insbesondere der G-/C-Gehalt und/oder die Basenpaarungswahrscheinlichkeiten eingesetzt.

Bei einer weiteren bevorzugten Ausführungsform erfolgt die Berechnung der Expressionseffizienz für jedes der Expressionskonstrukte durch wechselseitige Verknüpfung mit den in Schritt B) bestimmten Attributwerten mittels Computerlemender Verfahren, die einen Entscheidungsbaum aus einem Satz von Fällen, die zu bekannten Klassen gehören, aufbauen (J.R. Quinlan, C4.5: Programs for Machine Learning, Morgan Kaufinann, San Mateo, Kalifornien, USA 1993).

Bei einer weiteren bevorzugten Ausführungsform erfolgt die Berechnung der Expressionseffizienz für jedes der Expressionskonstrukte durch wechselseitige Verknüpfung mit den in Schritt B) bestimmten Attributwerten mittels neuronaler Netze (siehe z.B. D.E. Rumelhart et al, "Learning Representations by Back-Propagating Errors", Nature 1986, 323, 533-636; R. Hecht-Nielsen, "Theory of the Backpropagation Neural Network", in Neural Networks for Perception, pp. 65-93 (1992); D. Nauck, F. Klawonn, R. Kruse, Neuronale Netze und Fuzzy-Systeme, Vieweg-Verlag, 1994; Rüdiger Brause, Neuronale Netze, Teubner-Verlag 1995; R. Rojas, Theorie der Neuronalen Netze, Springer-Verlag, 1993; A. Zell, Simulation Neuronaler Netze; Addison-Wesley, 1994).

Bei einer weiteren bevorzugten Ausführungsform erfolgt die Berechnung der Expressionseffizienz für jedes der Expressionskonstrukte mittels Bayscher Netze (J. Pearl, Probabilistic Reasoning in Intelligent Systems, Morgan Kaufmann, San Mateo, Kalifornien, USA, 1988).

Bei einer weiteren bevorzugten Ausführungsform werden die Expressionskonstrukte sowie deren Proteinprodukte nach ungewünschten Fragmentierungsstellen analysiert und dementsprechend in Schritt A) Expressionskonstrukte generiert, mit denen die Fragmentierung minimiert werden kann. Beispiele für ungewünschte Fragmentierungsstellen innerhalb der kodierenden Sequenz sind interne Initiationsstellen, vorzeitige Terminationsstellen und/oder seltene Kodon-Cluster. Ungewünschte Fragmentierungsstellen innerhalb des Proteinprodukts sind insbesondere proteolytische Spaltstellen.

Bei einer speziellen Ausführungsform unter Verwendung von Expressionsvektoren umfasst das erfindungsgemäße Verfahren folgende Schritte:
a) Bereitstellung einer das herzustellende Protein kodierenden Nukleinsäuresequenz;
b) Spezifizierung von Randbedingungen betreffend die gewünschte Klonierungsstrategie, die Inkorporierung von Reinigungs- und/oder Detektions-Tags, und/oder die Anzahl bzw. Art von erlaubten Aminosäuresubstitutionen;
c) Auswahl eines geeigneten Expressionsvektors;
d) Generierung mindestens eines die für das Protein kodierende native Sequenz enthaltenden Expressionskonstrukts;
e) Generierung eines oder mehrerer veränderter Expressionskonstrukte durch Nukleotidsubstitutionen und/oder -insertionen und/oder -deletionen;
f) Berechnung der Expressionseffizienz für jedes der Expressionskonstrukte durch wechselseitige Verknüpfung mit mindestens einem die Expressionseffizienz beeinflussenden Attributwert, ausgewählt aus quantitativen Primärstrukturmerkmalen, qualitativen Primärstrukturmerkmalen und quantitativen Sekundärstrukturmerkmalen;
g) Ausgabe der für das/die Expressionskonstrukte berechneten Expressionseffizienzen.

Bei einer weiteren speziellen Ausführungsform unter Verwendung von PCR-generierten Matrizen umfasst das erfindungsgemäße Verfahren folgende Schritte:
a) Bereitstellung einer das herzustellende Protein kodierenden Nukleinsäuresequenz;
b) Spezifizierung von Randbedingungen betreffend die Inkorporierung von Reinigungs- und/oder Detektions-Tags; und/oder die Anzahl bzw. Art von erlaubten Aminosäuresubstitutionen;
c) Generierung mindestens eines die für das Protein kodierende native Sequenz enthaltenden Expressionskonstrukts;
d) Generierung eines oder mehrerer veränderter Expressionskonstrukte durch Nukleotidsubstitutionen und/oder -insertionen und/oder -deletionen;
e) Berechnung der Expressionseffizienz für jedes der Expressionskonstrukte durch wechselseitige Verknüpfung mit mindestens einem die Expressionseffizienz beeinflussenden Attributwert, ausgewählt aus quantitativen Primärstrukturmerkmalen, qualitativen Primärstrukturmerkmalen und quantitativen Sekundärstrukturmerkmalen;
f) Generierung von PCR-Primersequenzen;
g) Ausgabe der für das/die Expressionskonstrukte berechneten Expressionseffizienzen und/oder der PCR-Primersequenzen für die Expressionskonstrukte.

Die Generierung von PCR-Primersequenzen in Schritt f) erfolgt üblicherweise unter Berücksichtigung der dem Fachmann bekannten Regeln zum PCR-Primer-Design (Newton & Graham, PCR, Spektrumverlag, Heidelberg, Deutschland, 1994; McPherson & Moller, PCR, BIOS Scientific Publishers, Oxford, Großbritannien, 2000; Kain et al., 1991)

Die vorstehend genannten Schritte für die Ausführungsformen unter Verwendung von Expressionsvektoren bzw. unter Verwendung von PCR-generierten Matrizen, jeweils a) bis g), können einzeln oder in beliebiger Kombination und Reihenfolge durchgeführt werden. Der Fachmann ist in der Lage, ausgehend von dem ihm gestellten speziellen Problem eine geeignete Auswahl und Reihenfolge der vorstehend genannten Verfahrensschritte zu treffen.

Das erfindungsgemäße Verfahren kann gegebenenfalls ferner die Bereitstellung von Daten betreffend die physiko-chemischen Eigenschaften des Proteinprodukts, von Vorschlägen zu ihrer Verbesserung und/oder einer Anleitung der einzelnen Schritte zur Herstellung der Expressionskonstrukte umfassen.

Das vorstehend beschriebene erfindungsgemäße Verfahren zur Vorhersage der Expressionseffizienz wird vorzugsweise computergestützt durchgeführt. D.h. vorzugsweise werden mindestens einer und besonders bevorzugt alle Verfahrensschritte auf einem Computer wie beispielsweise einem PC durchgeführt werden. Bei einer speziellen Ausführungsform eines derartigen computergestützten Verfahrens wird die kodierende Nukleinsäuresequenz beispielsweise in Textformat bereitgestellt.

Ein weiterer Gegenstand der vorliegenden Erfindung betrifft ein computerlesbares Medium mit auf diesem gespeicherten computerausführbaren Anweisungen zum Ausführen des vorstehend beschriebenen erfindungsgemäßen Verfahrens.

Ein weiterer Gegenstand der vorliegenden Erfindung betrifft ein Computerprogramm-Produkt, dergestalt ausgebildet, dass bei einer Ausführung des Computerprogramm-Produkts auf einem Computer das vorstehend beschriebene erfindungsgemäße Verfahren ausgeführt wird.

Ein weiterer Gegenstand der vorliegenden Erfindung betrifft ein Verfahren zur Herstellung eines Proteins in, vorzugsweise prokaryontischen, zellulären Expressionssystemen, das die folgenden Schritte umfasst:
a) Vorhersage der Expressionseffizienz für Expressionskonstrukte gemäß dem vorstehend beschriebenen Verfahren;
b) Auswahl eines Expressionskonstrukts mit einer bestimmten, vorzugsweise der höchsten Expressionseffizienz;
c) Zelluläre Expression des Proteins basierend auf dem Expressionskonstrukt aus Schritt b).

Ein weiterer Gegenstand der vorliegenden Erfindung betrifft ein Verfahren zur Herstellung eines Proteins in, vorzugsweise prokaryontischen, zellfreien Expressionssystemen, das die folgenden Schritte umfasst:
a) Vorhersage der Expressionseffizienz für Expressionskonstrukte gemäß dem vorstehend beschriebenen Verfahren;
b) Auswahl eines Expressionskonstrukts mit einer bestimmten, vorzugsweise der höchsten Expressionseffizienz;
c) in vitro-Synthese des Proteins basierend auf dem Expressionskonstrukt aus Schritt b).

Im Folgenden werden spezielle Ausführungsformen des erfindungsgemäßen Verfahrens zur Vorhersage der Expressionseffizienz für Expressionslconstrukte beschrieben.

Die Generierung von einem, vorzugsweise mehreren Expressionskonstrukten für eine vorgegebene Nukleinsäuresequenz umfasst eine Reihe von Schritten. Die vorgegebene Nukleinsäuresequenz umfasst mindestens den Bereich, der das in dem prokaryontischen Expressionssystem zu exprimierende Protein kodiert. Die Sequenz kann Start- und Stop-Kodons je nach Art der Sequenz, d.h. beispielsweise eine vollständige oder eine Einzeldomänen-kodierende Sequenz, beinhalten. Die vorgegebene Sequenz kann gegebenenfalls nach Fehlern wie beispielsweise Doppeldeutigkeiten oder falschen Zeichen sowie hinsichtlich der Gegenwart von Start- und Stop-Kodons analysiert werden.

Wie bereits vorstehend erwähnt, können in Schritt A) des erfindungsgemäßen Verfahrens bei der Generierung des Expressionskonstrukts Randbedingungen und Präferenzen für die Expressionskonstrukte spezifiziert werden. Randbedingungen umfassen die geplante Klonierungsstrategie, spezielle Charakteristika des Expressionskonstrukts, beispielsweise die Position und Art eines Tags, sowie das Zulassen von Aminosäuresubstitutionen.

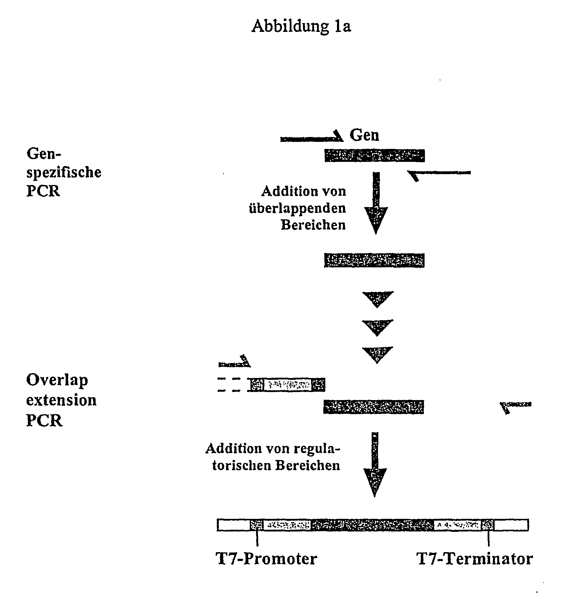

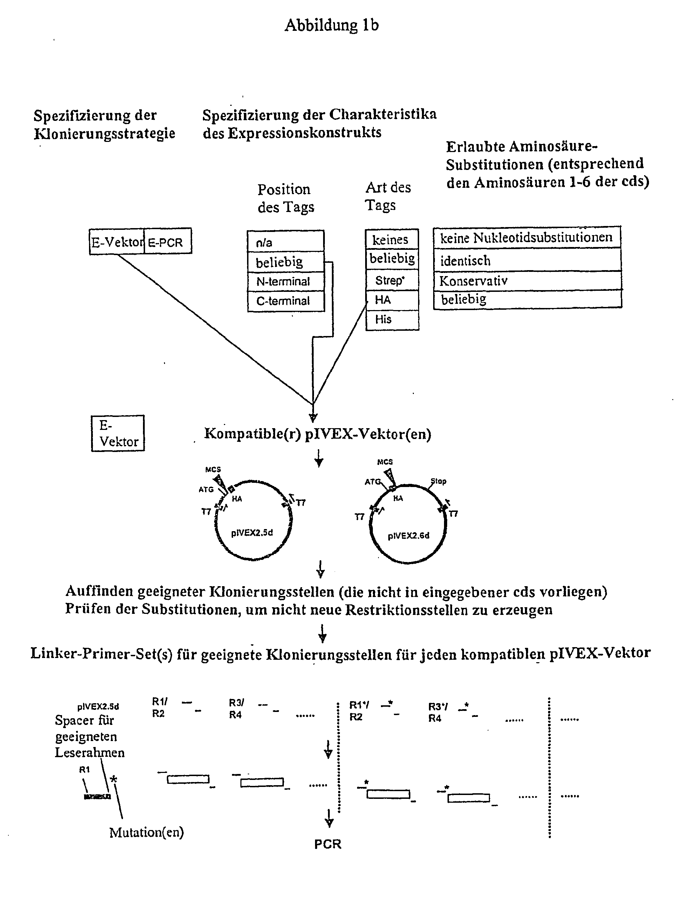

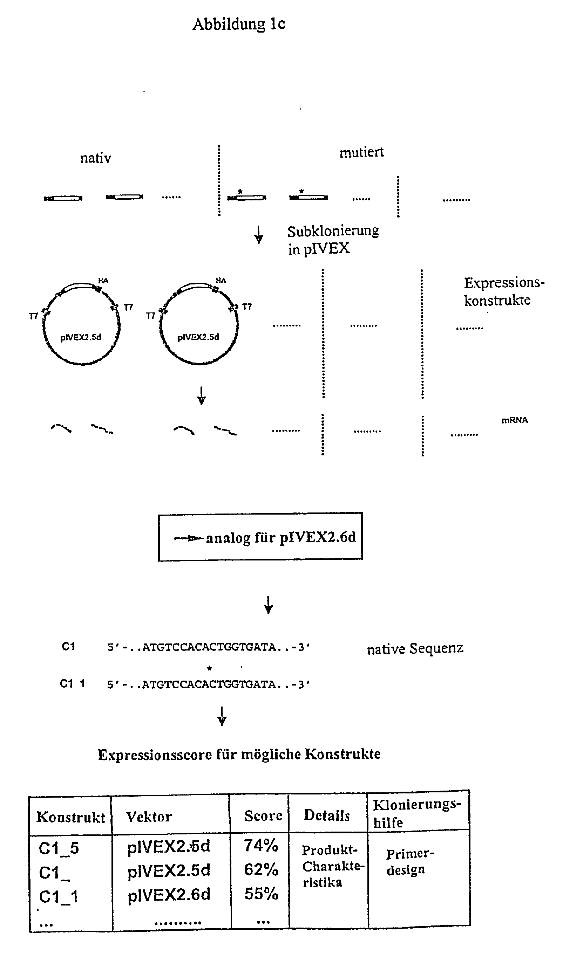

Das Expressionskonstrukt kann entweder als zirkuläres oder lineares Konstrukt spezifiziert und generiert werden (siehe Abbildung 1). Bei Verwendung zirkulärer Expressionskonstrukte schließt die Analyse die Identifizierung von geeigneten Restriktionsstellen für die Klonierung ein. Deswegen wird der ausgewählte Vektor, z.B. pIVEX (Roche Diagnostics GmbH, Mannheim), nach Restriktionsstellen entweder in der vielfachen Klonierungsstelle oder an anderen Stellen abgesucht, die nicht in der gegebenen kodierenden Sequenz enthalten sind (siehe Abbildung 1b). Ferner können Spacer-Sequenzen enthaltende Linker-Primer entwickelt werden, um die Translation in dem gewünschten Leserahmen sicher zu stellen.

Bei der Generierung der Expressionskonstrukte in Schritt A) des erfindungsgemäßen Verfahrens können ferner verschiedene Arten von Tags ausgewählt werden, falls diese bei der Synthese erwünscht sind. Im Rahmen der vorliegenden Erfindung geeignete Tags sind unter anderem solche, die herkömmlich für die Reinigung oder Detektion des Expressionsprodukts verwendet werden. Beispiele hierfür sind Streptag, Hexa-Histidin-Tag oder HA-Tag (siehe Tabelle 1). Ferner kann der Ort des Tags, beispielsweise C-terminal oder N-terminal ausgewählt werden. Alternativ kann bei der Generierung des Expressionskonstrukts auch zwischen verschiedenen Fusionskonstrukten, beispielsweise dem Maltose-bindendes Protein (MBP) und Glutathion-S-Transferase (GST), ausgewählt werden.

**Tabelle 1: Expressionsklonierungsvektoren pIVEX, die mit dem zellfreien E. coli-Expressionssystem RTS Expert (Roche Diagnostics) kompatibel sind, sowie deren Charakteristika.**

| Klonierungsvektor | Klonierungsstellen | Faktor Xa Restriktionsproteasespaltstelle für das Tag | Art des Tags | Position des Tags |
|---|---|---|---|---|
| pIVEX 2.3d | MCS | - | His | C-terminal |
| pIVEX 2.4d | MCS | X | His | N-terminal |
| pIVEX MBP | MCS | X | His + MBP | N-terminal |
| pIVEX 2.5d | MCS | - | HA | C-terminal |
| pIVEX 2.1d | MCS | - | Streptag | C-terminal |
| pIVEX 2.2d | MCS | - | Streptag | N-terminal |
| pIVEX-GST | MCS | - | His+GST | N-terminal |
| pIVEX 2.6d | MCS | X | HA | N-terminal |

Wie bereits vorstehend beschrieben, können alternative Nukleotidsequenzen generiert werden, um die Expressionseffizienz der resultierenden Konstrukte zu verbessern. Die alternativen Sequenzen werden vorzugsweise ebenfalls hinsichtlich der Gegenwart von Restriktionsstellen innerhalb der kodierenden Sequenz analysiert.

Eine Vektor-basierte Expression erfordert zunächst die Auswahl desjenigen Vektors, der die angegebenen Randbedingungen erfüllt. Im weiteren ist es vorteilhaft, alle Expressionskonstrukte zu generieren, die sich aus der Kombination der potentiellen Einklonierungsstellen in Verbindung mit den festgelegten Randbedingungen ergeben.

Lineare Expressionskonstrukte können über verschiedene PCR-Techniken hergestellt werden. Dabei werden die für die Expression nötigen regulatorischen Elemente entweder über lange äußere Primer oder mittels zugegebener DNA und overlap-extension PCR anfusioniert (Newton & Graham, 1994, siehe oben; McPherson and Moller, 2000, siehe oben; Kain et al., 1991, siehe oben). Letztere Methode ist im RTS Linear Template Generation Set von Roche Diagnostics realisiert. Dabei werden in einem ersten PCR-Schritt überlappende Sequenzen an die Protein-kodierende Sequenz anfusioniert. In einem zweiten PCR-Schritt werden über die Überlappungsbereiche mittels overlap-extension die regulatorischen Regionen eingeführt (siehe Abbildung 1a). Dabei können analog zu den zirkulären Expressionskonstrukten Tagsequenzen oder Fusionsproteinsequenzen eingeführt werden.

Zur Generierung von linearen Expressionskonstrukten werden vorzugsweise in Schritt A) des erfindungsgemäßen Verfahrens Mutationen im kodierenden Bereich der Gen-spezifischen ersten Primer vorgenommen. Des weiteren können auch Mutationen im regulatorischen Bereich eingeführt werden.

Unabhängig von der ausgewählten Strategie zur Herstellung von Expressionskonstrukten bietet das erfindungsgemäße Verfahren die Möglichkeit, für jedes Expressionskonstrukt detaillierte Charakteristika der mRNA und des Translationsproduktes anzugeben.

Insbesondere bei der computergestützten Durchführung des erfindungsgemäßen Verfahrens können eine Liste der Expressionskonstrukte mit der jeweils berechneten Expressionsausbeute sowie Verknüpfungen zu erweiterten mRNA- und ProteinCharakteristika und/oder spezifische Klonierungshilfen bereitgestellt werden. Vorzugsweise werden die Expressionskonstrukte entsprechend den zu erwartenden Expressionsmengen angeordnet. Konstrukte, die von mutierten kodierenden Sequenzen abgeleitet sind, werden nur angezeigt, wenn eine höhere Expressionsausbeute zu erwarten ist als mit Konstrukten der nativen Sequenz.

Bei einer weiteren bevorzugten Ausführungsform der vorliegenden Erfindung können in Schritt A) des erfindungsgemäßen Verfahrens Mutationen generiert werden. Zur Erzeugung von Mutationen bzw. Deletionen und/oder Insertionen eignet sich insbesondere der Anfangsbereich in einer translatierten Region, da die Expressionsmenge durch die Sequenz in diesem Bereich stark beeinflusst wird. Somit werden zur Verbesserung der Expression vorzugsweise Mutationen in den ersten Kodons der translatierten Region, besonders bevorzugt den ersten 60 Nukleotiden, mehr bevorzugt den ersten 30 Nukleotiden und am meisten bevorzugt den ersten 21 Nukleotiden, inklusive Start-Kodon, generiert. Die Mutationen können selbstverständlich auch regulatorische Sequenzen upstream des Start-Kodons betreffen.

Die Mutationen werden insbesondere nach folgenden Mutations-Generierungsregeln ausgewählt, die Mutationen bevorzugen, welche einen positiven Effekt auf die vorhergesagte Expressionsmenge haben: So ist die Anzahl an Kodons der kodierenden Sequenz nach dem Start-Kodon, die durch Mutationen verändert wird, vorzugsweise höchstens zehn, besonders bevorzugt höchstens acht und am meisten bevorzugt höchstens 6. Der Kodonadaptionsindex für jedes Kodon ist ferner vorzugsweise mindestens 0,02, besonders bevorzugt mindestens 0,05 und am meisten bevorzugt mindestens 0,07, während der G/C-Gehalt für jedes Kodon insbesondere höchstens 0,7 und besonders bevorzugt höchstens 0,4 ist.

Ferner kann bei Schritt A) des erfindungsgemäßen Verfahrens bei der Generierung des Expressionskonstrukts gewählt werden, welche Art von Aminosäuresubstitutionen durch die eingeführten Nukleinsäuresubstitutionen gestattet werden. Dabei bestehen insbesondere die folgenden drei Möglichkeiten: Nukleotidsubstitutionen, die zu identischen Aminosäuren führen; Nukleotidsubstitutionen, die zu konservativen Aminosäureaustauschen führen, sowie Nukleotidsubstitutionen, die zu einer beliebigen Aminosäure führen.

In Abhängigkeit von den vorstehend genannten Parametern im Zusammenhang mit der Auswahl des Expressionskonstrukts und der Erzeugung von Mutationen kann somit für eine einzelne gegebene kodierende Sequenz eine Vielzahl von Expressionskonstrukten bereitgestellt werden, für die jeweils die Expressionsmengen mit dem erfindungsgemäßen Verfahren vorhergesagt werden können.

Im Schritt B) des erfindungsgemäßen Verfahrens zur Vorhersage der Expressionseffizienz werden die die Expressionseffizienz beeinflussenden Attributwerte der kodierenden Sequenz bestimmt. Derartige Attributwerte der kodierenden Sequenz können ausgewählt werden aus quantitativen Primärstruktur-Attributen, qualitativen Primärstruktur-Attributen und/oder quantitativen Sekundärstruktur-Attributen. Besonders bevorzugte Attribute, nach denen die gegebene DNA-Sequenz bzw. das die kodierende Sequenz umfassende Expressionskonstrukt analysiert wird, sind der G/C-Gehalt von Teilbereichen oder dem gesamten Bereich der kodierenden Sequenz, die erste Base des zweiten Kodons der kodierenden Sequenz, die Basenfolge des zweiten Kodons und/oder die Basenpaarungswahrscheinlichkeiten für mindestens eine der Basen der kodierenden Sequenz, vorzugsweise innerhalb der ersten 60 Basen, besonders bevorzugt innerhalb der ersten 21 Basen.

In Schritt C) des erfindungsgemäßen Verfahrens wird schließlich die Expressionseffizienz für jedes der Expressionskonstrukte berechnet, indem die in Schritt B) bestimmten Attributwerte verknüpft werden. Anhand der daraus berechneten Expressionseffizienz bzw. -menge kann das vielversprechendste Expressionskonstrukt ausgewählt werden. Für die Verknüpfung der Attributwerte ist für jede gegebene kodierende Sequenz eine Vorhersage-Verknüpfung erforderlich.

Vorzugsweise wird eine derartige Vorhersage-Verknüpfung aus der Abhängigkeit experimentell ermittelter Expressionsausbeuten (im Folgenden auch als Trainingsdaten bezeichnet) von Attributwerten der dabei eingesetzten Expressionskonstrukte abgeleitet. Vorzugsweise werden dazu Expressionsausbeuten von mindestens 100, besonders bevorzugt von mindestens 500 und am meisten bevorzugt von mindestens 1000 Sequenzen experimentell ermittelt. Bei weiteren Ausführungsformen werden die Expressionsausbeuten von mindestens 20, mindestens 50, mindestens 250, mindestens 750 oder mindestens 900 Sequenzen experimentell ermittelt. Die derart einmal basierend auf einem Satz von Trainingsdaten abgeleitete Vorhersage-Verknüpfung bzw. der daraus abgeleitete Vorhersage-Algorithmus kann dann zur Berechnung der Expressionseffizienz für in Schritt A) des erfindungsgemäßen Verfahrens generierte Expressionskonstrukte angewendet werden.

Bei einer besonderes bevorzugten Ausführungsform wird die Vorhersage-Verknüpfung aus der Abhängigkeit von für ein bestimmtes Expressionssystem experimentell ermittelten Expressionsausbeuten von Attributwerten der dabei eingesetzten Expressionskonstrukte abgeleitet. Die Vorhersage-Verknüpfung wird dann zur Berechnung der Expressionseffizienz für solche in Schritt A) des erfindungsgemäßen Verfahrens generierte Expressionskonstrukte angewendet, die mit dem Expressionssystem kompatibel sind.

Bei einer weiteren besonders bevorzugten Ausführungsform wird zur Erzeugung der Trainingsdaten der nicht-translatierte, 5' des Startkodons liegende Bereich (5'-UTR, 5' untranslated region) unverändert gelassen. Somit wird die Vorhersage-Verknüpfung bei dieser Ausführungsform aus der Abhängigkeit experimentell ermittelter Expressionsausbeuten von Attributwerten der dabei eingesetzten Expressionskonstrukte abgeleitet, wobei die Expressionskonstrukte lediglich Unterschiede in den Basen beginnend mit dem zweiten Kodon der kodierenden Sequenz, d.h. im translatierten Bereich aufweisen. Vorzugsweise werden dabei Sequenzen analysiert, die Unterschiede in den 150 Basen, besonders bevorzugt in den 90 Basen und am meisten bevorzugt in den 39 Basen beginnend mit dem zweiten Kodon der kodierenden Sequenz aufweisen. Es können allerdings auch Sequenzen analysiert werden, die Unterschiede in den 120 Basen, den 60 Basen oder den 30 Basen beginnend mit dem zweiten Kodon der kodierenden Sequenz aufweisen.

Basierend auf einer wie vorstehend beschriebenen Ableitung der Vorhersage-Verknüpfung aus experimentell ermittelten Daten kann die Translationseffizienz vom mRNA-Sequenzen mit hoher Genauigkeit vorhergesagt werden.

Die Datenanalyse der Trainingsdaten wird detailliert anhand eines Beispiels beschrieben.

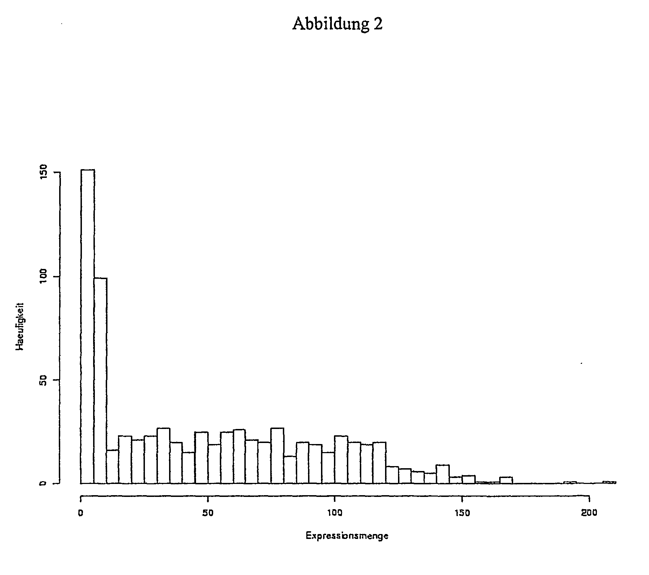

Im Folgenden wird ein Überblick über die wesentlichen Punkte zur Bereitstellung des Vorhersage-Verknüpfung aus Trainingsdaten gegeben. So wurden beispielsweise zur Bereitstellung einer Datenbasis die experimentellen Expressionsdaten von 742 Sequenzen analysiert, die lediglich Unterschiede in den 39 Basen beginnend mit dem zweiten Kodon der kodierenden Sequenz aufwiesen. Abbildung 2 zeigt ein Histogramm der Expressionsmengen, die durch in vitro-Expression dieser Sequenzen erhalten wurden. Die Expressionsmenge wird als prozentualer Anteil an der Expression des mit den vorstehend kodierenden Sequenzen fusionierten GFP-Proteins angegeben. Obwohl die variable Region relativ kurz ist, wird ein breite Verteilung an Expressionswerten beobachtet.

Für jede der Sequenzen wurde eine Reihe an Attributwerten ausgewertet. Die die Expressionsmenge beeinflussenden Attributwerte können beispielsweise durch Korrelationsanalyse oder Histogramme identifiziert werden. Als wichtigste Sequenzattribute haben sich der GC-Gehalt und die Basenpaarungswahrscheinlichkeiten innerhalb der ersten 20 bis 40 Basen der kodierenden Sequenz erwiesen. Dies gilt insbesondere für den hier untersuchten speziellen Trainingsdatensatz, welcher im Translationsinitiationsbereich keine Sequenzvariabilität aufweist. Im Allgemeinen hat die Sequenz des Initiationsbereiches einen großen Einfluss auf die Menge des exprimierten Proteins.

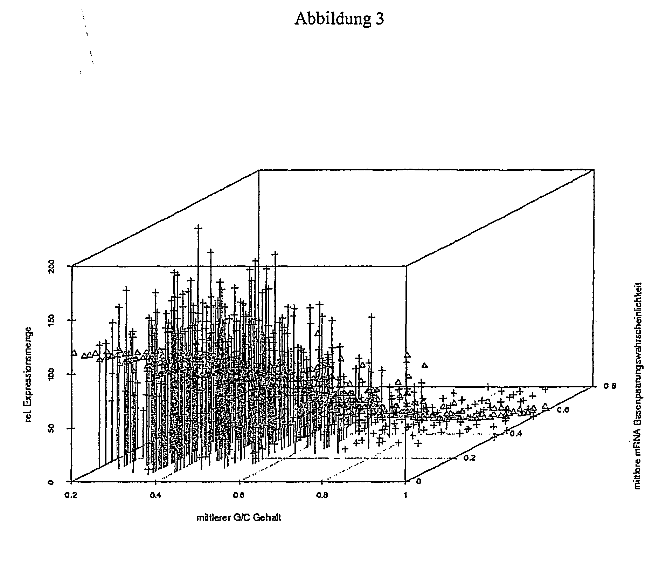

Zur Erzeugung einer Vorhersage-Verknüpfung wird vorzugsweise eine herkömmliche Regressionsanalyse verwendet. So können beispielsweise der GC-Gehalt und die Basenpaarungswahrscheinlichkeit, beide gemittelt über einen Bereich downstream von dem Translationsstartkodon, als unabhängige Variable zur Anpassung des beobachteten Expressionslevels verwendet werden (siehe Abbildung 3).

Alternativ kann mit Hilfe von Maschinenlernverfahren ein Entscheidungsbaum abgeleitet werden, welcher ebenfalls eine Vorhersage der Expressionsmenge erlaubt.

Um die Verlässlichkeit der Vorhersage der in dem erfindungsgemäßen Verfahren verwendeten wechselseitigen Verknüpfung mit den in Schritt B) bestimmten Attributwerten zu bestimmen, können die vorhergesagten und experimentell gemessenen Expressionswerte für einen gegebenen Datensatz verglichen werden. Die Abweichung zwischen erwarteten und beobachteten Werten wird in einem Histogramm für den Datensatz aufgezeichnet, aus dem das Regressionsmodell erhalten wurde.

Alternativ wird als Trainingsdatensatz nur ein Teil des gesamten Datensatzes verwendet. Die Abweichungen der vorhergesagten und der experimentell gemessenen Expressionsmenge werden für den Rest des Datensatzes, der nicht als Trainingsdatensatz verwendet wurde, in einem Histogramm dargestellt.

Ein weiterer Weg zur Beurteilung der Vorhersagegenauigkeit ist die Bestimmung der Anzahl von richtigen Positiven (d.h. positive Expression und positive vorhergesagte Expression), falschen Positiven (d.h. negative Expression und positive vorhergesagte Expression), richtigen Negativen (d.h. negative Expression und negativ vorhergesagte Expression) und falschen Negativen (d.h. positive Expression und negativ vorhergesagte Expression). Positive bzw. negative Expression bedeutet in diesem Zusammenhang, dass die Expressionsmenge über bzw. unter einem bestimmten Schwellenwert, vorzugsweise angegeben in Referenzexpressionseinheiten, liegt. Beispielsweise kann der Schwellenwert eine relative Expressionsmenge von 0,30 REE sein. Die Genauigkeit der Vorhersage bestimmt sich aus der Summe aus richtigen Positiven und richtigen Negativen, d.h. aller richtig vorhergesagten Fälle, dividiert durch die Summe aller vorhergesagten Fälle.

Bei der vorstehend dargestellten Vorgehensweise zur Erzeugung einer Vorhersage-Verknüpfung wird von einem Datensatz ausgegangen, bei dem die Sequenzen nur in den ersten 39 Nukleotiden downstream von dem Translationsstartkodon variieren. Diese Region wurde im Rahmen der vorliegenden Erfindung als besonders wesentlich für die Translationseffizienz identifiziert. Selbstverständlich können auch Trainingsdaten verwendet werden, bei dem die Sequenzen in einem größeren Bereich downstream von dem Translationsstartkodon variieren, beispielsweise in einem Bereich von jeweils 40 Basen upstream und downstream vom Translationsstartkodon variieren. Des weiteren können auch größere Bereiche, beispielsweise innerhalb der ersten 50, 75 oder 100 oder noch mehr Nukleotiden upstream und/oder downstream von dem Translationsstartkodon variiert werden.

Ferner können auch Trainingsdaten verwendet werden, bei denen weitere Attribute bestimmt werden, die in den in dem vorstehend beschriebenen Datensatz konstant gehaltenen Regionen vorliegen. Eine derartige Datenbank aus Trainingsdaten ist umso besser geeignet für die Erzeugung einer präzisen Vorhersage-Verknüpfung, je höher die Variabilität in den verschiedenen Attributen, wie beispielsweise der Länge der variierten Sequenz, der Länge der kodierenden Sequenz, der Länge der mRNA-Sequenz, der Kodonadaptionsindizes und dergleichen, ist.

Bei einer weiteren Ausführungsform des erfindungsgemäßen Verfahrens werden in einem zusätzlichen Verfahrensschritt die gegebene Nukleotidsequenz sowie die abgeleitete Aminosäuresequenz hinsichtlich kritischer Stellen, die zu einer Produktfragmentierung führen, analysiert. Ferner kann eine Charakterisierung des Produkts hinsichtlich seiner biochemischen und funktionellen Eigenschaften durchgeführt werden. In Abhängigkeit von den daraus erhaltenen Ergebnissen können Alternativen für eine Verbesserung der Translationsergebnisse bereitgestellt werden.

Häufig führt die in vitro-Expression zu ungewünschten Fragmentierungen von bestimmten Proteinen. Derartige Fragmentierungen können aufgrund von unterschiedlichen sequenzspezifischen Mustern der mRNA oder des Proteins auftreten, die entweder zu einer nicht vollständigen Translation oder zu einem proteolytischen Abbau führen. Derartige Sequenzen können bei dieser Ausführungsform des erfindungsgemäßen Verfahrens identifiziert werden, wobei vorzugsweise gleichzeitig Vorschläge für die Minimierung von derartigen Fragmentierungen und für die Erhöhung der Ausbeute eines vollständigen Produkts bereitgestellt werden können.

So kann beispielsweise eine Produktfragmentierung auftreten, wenn interne Translationsstartstellen in der kodierenden Sequenz vorliegen. Beispiele hierfür sind interne Shine-Dalgarno-artige Sequenzen in der Nähe von möglichen Initiationskodons, die durch *E. coli*-Ribosomen als alternative Translationsinitiationsstellen erkannt werden können. *E. coli*-Initiationskodons sind AUG (91%), GUG (8%) oder UUG (1%) (Makrides, 1996). Derartige kritische Stellen werden häufiger in eukaryontischen Genen gefunden, da Shine-Dalgarno-Stellen für die eukaryontische Translation nicht notwendig sind und deswegen nicht durch die Evolution eliminiert wurden.

Kritische Sequenzkonstellationen sind besonders dann von besonderer Bedeutung für die Expressionsausbeute, wenn der eigentliche Startkodon schlecht zugänglich ist, beispielsweise wenn sich der AUG-Startkodon in einer Region befindet, die stabile mRNA Sekundärstrukturen ausbildet.

Somit werden bei einer bevorzugten Ausführungsform des erfindungsgemäßen Verfahrens die gegebene kodierende Sequenz hinsichtlich derartiger Muster, die eine Fragmentierung während der Expression verursachen können, analysiert. Wenn ein derartiges Sequenzmuster gefunden wird, werden Empfehlungen für die Verbesserung der Sequenz gegeben, die bei der Generierung des Expressionskonstrukts in Schritt A) des erfindungsgemäßen Verfahrens berücksichtigt werden können.

Ferner ist bekannt, dass stabile interne β-Strukturen in der mRNA zu einer unvollständigen Translation führen können (de Smit MH, van Duin J.; J. Mol. Biol. 1994, 235, 173-184). Folglich kann basierend auf Regeln für die Vorhersage derartiger Strukturen anhand der gegebenen Sequenz eine entsprechende Verknüpfung zur Bestimmung derartiger Strukturen entwickelt werden und ebenfalls bei der Generierung von Expressionskonstrukten berücksichtigt werden.

Ein weiterer Faktor, der die Genexpression stark beeinflusst und in dem erfindungsgemäßen Verfahren in Schritt A) bei der Generierung von Expressionskonstrukten vorzugsweise berücksichtigt wird, ist die selektive Verwendung von Kodons in bestimmten Wirtsorganismen. Im Allgemeinen enthalten Gene, die mit geringer Häufigkeit exprimiert werden, eine größere Anzahl an seltenen Kodons als hochexprimierte Gene. Die Verwendung von Kodons in einem bestimmten Gen bezogen auf die allgemeine Verwendung dieser Kodons in exprimierten Genen eines Wirtsorganismus wird als Kodonadaptionsindex bezeichnet (CAI, Sharp and Li, Nucleic Acids Res. 1987, 15, 1281-1295). Da der Kodonadaptionsindex Spezies-spezifisch ist, sollte die Kodonverwendung für das zu exprimierende Gen möglichst der des Wirts entsprechen. In einem zellfreien System wird entsprechend die Kodonverwendung in dem Organismus berücksichtigt, aus dem das bei der in vitro-Expression eingesetzte Zell-Extrakt stammt.

D.h. um eine maximale Genexpression zu gewährleisten, sollte der Kodonadaptionsindex möglichst hoch sein, vorzugsweise mindestens 0,05. Beispielsweise können die Expressionsausbeuten aus Säugergenen in *E*. *coli* gering sein, da dort häufiger Argininkodons AGG und AGA auftreten, für die es in *E*. *coli* nur eine geringe Konzentration an entsprechenden tRNAs gibt. Vielfach ist es so, dass die Expression signifikant steigt, wenn seltene Kodons durch häufig in einem Organismus verwendete Kodons ersetzt werden (siehe Makrides, 1996).

Die Korrelation von seltenen Kodons mit einer geringen Expressionsausbeute ist besonders signifikant, wenn sich die Kodons am Anfang der 5'-Region der mRNA, insbesondere innerhalb der ersten 25 Kodons des Gens, befinden. Lokale Cluster von seltenen Kodons können ferner zu frame shifts führen und in manchen Fällen eine vorzeitige Termination aufgrund einer abnormalen Translationspause verursachen.

Somit wird bei einer weiteren bevorzugten Ausführungsform des erfindungsgemäßen Verfahrens in Schritt A) bei der Generierung eines Expressionskonstrukts die das herzustellende Protein kodierende Sequenz, insbesondere die ersten 25 Kodons, hinsichtlich seltener Kodons analysiert und die Abweichung zwischen der Kodonverwendung der zu exprimierenden Sequenz und dem gewünschten Kodonadaptionsindex bestimmt. Basierend auf einer eventuellen Abweichung können Vorschläge für konservative Basensubstitutionen gemacht werden, um die Kodons an häufig in dem Wirtsorganismus verwendete Kodons anzupassen. Denkbar sind beispielsweise auch nicht konservative Basensubstitutionen, die zu häufig in dem Wirtsorganismus verwendeten Kodons führen und konservative Aminosäureaustausche ergeben. Diese konservativen oder nicht konservativen Basensubstitutionen werden vorzugsweise bei der Generierung des Expressionskonstrukts in Schritt A) des erfindungsgemäßen Verfahrens berücksichtigt.

Alternativ kann vorgeschlagen werden, entsprechende tRNAs für die seltenen Kodons bei der Expressionsreaktion zuzugeben.

Eine weitere Ausführungsform des erfindungsgemäßen Verfahrens betrifft kritische Spaltstellen in dem herzustellenden Protein. Das Auftreten von proteolytischem Abbau, verursacht durch proteolytische Enzyme in dem zellfreien Lysat, beispielsweise aus *E. coli,* kann zu fragmentierten Proteinprodukten führen und stellt ein wesentliches Problem bei der Steigerung der Expressionsmenge an vollständigem Proteinprodukt dar. Die translatierten Polypeptide können insbesondere am N-Terminus Aminosäuren enthalten, die als proteolytische Spaltstellen erkannt werden.

Bei einer weiteren bevorzugten Ausführungsform des erfindungsgemäßen Verfahrens wird somit die translatierte Sequenz des durch die vorgegebene DNA-Sequenz kodierten Proteins hinsichtlich derartiger Spaltstellen analysiert, wobei vorzugsweise die entsprechenden Proteasen des Wirts berücksichtigt werden. So kann beispielsweise für *E. coli*-Proteasen eine nahezu vollständige Liste aus der Datenbank SWISS-PROT erzeugt werden (siehe Tabelle 2). Die Spezifitäten sind dem Fachmann aus der wissenschaftlichen Literatur bekannt.

Wenn die Art der Spaltung nicht charakterisiert werden konnte, wurde die Protease nicht berücksichtigt, selbst wenn gezeigt wurde, dass sie einen Abbau von heterolog exprimierten Proteinen verursachten, wie z.B. die Ion-Protease.

Einige der in Tabelle 2 gezeigten Proteasen können verschiedene Spezifitäten haben. Die bevorzugten Spaltstellen (Pn = N-Terminal, Pn' = C-Terminal) wurden gemäß der Schechter-und-Berger-Konvention angegeben (I. Schlechter und A. Berger, Biochem. Biophys. Res. Commun. 1967, 2:157-162). Aminosäuren werden unter Verwendung des Einbuchstabencodes angezeigt, Xaa steht für eine beliebige Aminosäure.

**Tabelle 2: Cytoplasmische Proteasen, die potentiell in einem E. coli-Lysat enthalten sind.**

| Enzym | Typ | P1 | P2 | P1' | P2' |
|---|---|---|---|---|---|
| Membranalanylaminotransferase pepN [EC 3.4.11.2] | Aminopeptidase | A | - | Xaa | Xaa |
| Membranalanylaminotransferase pepN [EC 3.4.11.2] | (Dipeptidylpeptidase) | P | A,V,L, I,P,W, F,M | Xaa | Xaa |
| Prolylaminopeptidase [EC 3.4.11.5] | Aminopeptidase | P | - | Xaa | Xaa |
| X-Pro-Aminopeptidase APP-II [EC 3.4.11.9] | Aminopeptidase | Xaa | - | P | Xaa |
| Bakterielle Leucylaminopeptidase [EC 3.4.11.10] | Aminopeptidase | L | - | Xaa | Xaa |
| Methionylaminopeptidase MAP [EC 3.4.11.18] | Aminopeptidase | M | - | Xaa | Xaa |
| Alanincarboxypeptidase [EC 3.4.17.6] | Carboxypeptidase | Xaa | Xaa | A | - |
| β-Aspartyl-peptidase [EC 3.4.19.5] | Aminopeptidase | D | - | Xaa | Xaa |
| Omptin ompT [EC 3.4.21.87] | Endopeptidase (Serine) | K | Xaa | R | Xaa |
| Pitrilysin Pi [EC 3.4.24.55] | Endopeptidase [Metallo] | Y | Xaa | L | Xaa |
| Pitrilysin Pi [EC 3.4.24.55] | Endopeptidase (Metallo) | F | Xaa | Y | Xaa |

Die Klassifizierung der ausgewählten Enzyme bezieht sich auf die NC-IOBMB-Peptidase-Nomenklatur (http://www.chem.qmw.ac.uk/iubmb/enzyme/ec34/).

Für eine in dem zellfreien Expressionssystem zu exprimierende Nukleotidsequenz können bei einer bevorzugten Ausführungsform des erfindungsgemäßen Verfahrens die Positionen der proteolytischen Erkennungsstellen in der resultierenden Aminosäuresequenz identifiziert werden und die entsprechenden Fragmentgrößen berechnet werden.

Ferner werden vorzugsweise Informationen über spezifische Inhibitoren und metallische Co-Faktoren, die Substratspezifität, spezifische Aktivität, optimale Temperatur und pH-Werte, KN-Wert, Stabilität, etc. der möglichen Proteasen bereitgestellt. Protease-spezifische Inhibitoren können beispielsweise mit Inhibitoren verglichen werden, die eine breite Spezifität aufweisen, um somit ungewünschte Nebenreaktionen zu vermeiden. Die Verwendung von Proteaseinhibitoren mit breiter Spezifität kann ferner andere Enzyme wie Methionylaminopeptidasen inaktivieren, die essentiell für die Entfernung des Start-Methionins in etwa einer Hälfte aller bakteriellen Proteine sind.

Des Weiteren werden bei einer weiteren Ausführungsform basierend auf der Analyse von potentiellen Spaltstellen Vorschläge für Basensubstitutionen gemacht, die konservative oder andere Aminosäureaustausche ergeben und zu einer Vermeidung von proteolytischen Erkennungsstellen in dem herzustellenden Protein führen. Derartige Basensubstitutionen werden vorzugsweise bei der Generierung des Expressionskonstrukts in Schritt A) des erfindungsgemäßen Verfahrens berücksichtigt.

Um hohe Ausbeuten des Proteinprodukts möglichst unter vollständigem Erhalt aller seiner Funktionen zu erzielen, ist es von besonderer Bedeutung, die Charakteristika des Proteins, insbesondere dessen physiko-chemische Eigenschaften zu kennen. Somit werden zur Vorbereitung einer optimalen in vitro-Synthese bei einer besonders bevorzugten Ausführungsform des erfindungsgemäßen Verfahrens basierend auf der kodierenden Nukleotidsequenz bzw. der Aminosäuresequenz des Proteins allgemeine Informationen über Länge, Molekulargewicht, isoelektrischen Punkt und dergleichen, sowie detaillierte Informationen über die zu erwartende Löslichkeit und Chaperon-Abhängigkeit bereitgestellt. Die vorstehend genannten und weitere Proteincharakteristika können bei der Planung der in vitro-Proteinsynthese berücksichtigt werden.

Die Ausbeute von Proteinen mit vollständiger Funktion hängt häufig von der Löslichkeit und für manche Proteine von Chaperonen ab. Chaperone sind Proteine, die durch Direktion der Faltung die korrekte Anordnung eines Targetproteins in die funktional aktive Konformation vermitteln. Die Expression von eukaryontischen rekombinanten Proteinen beispielsweise in einem *E. coli*-Expressionssystem führt häufig zur Akkumulation von unlöslichen Proteinaggregaten oder "inclusion bodies". Die Renaturierung der biologisch aktiven Produkte aus diesen aggregierten Konformationen ist für viele Polypeptide unmöglich, so beispielsweise für strukturell komplexe oligomere Proteine und solche Proteine, die eine Reihe von DisulfidBindungen enthalten.

Die Proteinlöslichkeit bzw. die Fähigkeit eines Proteins, Aggregate zu bilden, wird stark durch dessen Hydrophobizität und richtige Faltung bestimmt. So wird die Löslichkeit durch Cluster von hydrophoben Aminosäuren innerhalb des Polypeptids, beispielsweise transmembranen Domänen oder Signalpeptiden vermindert. In vivo-Chaperon-Systeme wie GroEL/GroES in *E. coli* katalysieren die komplexe Faltung von hydrophoben Resten von der Aggregations-anfälligen Proteinoberfläche in die inneren Regionen. Somit vermeiden Chaperone die falsche Selbstanordnung, die in vivo bei vielen Proteinen durch inter- oder intramolekulare Wechselwirkungen aufgrund von hydrophoben Stellen auftritt. Des Weiteren ist die Löslichkeit eines richtig gefalteten Proteins verglichen zu der nicht gefalteten Konformation stark erhöht.

Anhand der Aminosäuresequenz des durch die vorgegebene DNA-Sequenz kodierten Proteins werden bei einer Ausführungsform der erfindungsgemäßen Verfahren die zu exprimierenden Proteinsequenzen bezüglich Clustern von hydrophoben Aminosäuren und transmembranen Domänen analysiert und somit ihre Löslichkeit vorhergesagt. Für die Vorhersage von transmembranen Domänen kann beispielsweise der ALOM2-Algorithmus verwendet werden (P. Klein et al., Biochem. Biophys. Acta 1984, 787: 221-226). Aus diesen Ergebnissen können Rückschlüsse auf die mögliche Anordnung des Proteinprodukts, z.B. cytosolisch, membranständig und dergleichen, gezogen werden. Falls sich das zu exprimierende Protein als relativ unlöslich erweist, können entsprechende Vorschläge für die Planung der in vitro-Synthese, wie beispielsweise die Zugabe von milden Detergentien, die Erniedrigung der Reaktionstemperatur oder die Zugabe von Chaperonen, gemacht werden.

Obwohl sich Proteine spontan in ihrer nativen Struktur falten können, können Chaperone die Effizienz der Faltung durch Vermeidung von Aggregation und falscher Faltung verbessern. Ein besonders bevorzugtes Chaperon-System ist das GroEL/GroES-System aus *E. coli,* das unter allen Wachstumsbedingungen wirksam ist. In zellfreien Translationssystemen wurde gezeigt, dass die Zugabe mit GroEL/GroES die native Faltung von naszierender Aspartataminotransferase erleichtert (J.R. Matingly et al., Arch. Biochem. Biophys. 2000, 382:113-122). Neuere Studien haben gezeigt, dass etwa 300 neu translatierte Polypeptide mit unterschiedlicher Funktion stark mit GroEL wechselwirken und bei etwa einem Drittel die Aufrechterhaltung der Konformation direkt von dem Chaperon abhängt (W.A. Houry et al., Nature 1999, 402:147-54).

GroEL-Substrate enthalten vorzugsweise mindestens zwei α/β-Domänen, die eingeschlossene β-Faltblätter mit großen hydrophoben Oberflächen umfassen und haben eine Molekularmasse von vorzugsweise Mᵣ = 20-60 K.

Basierend auf den vorstehend dargestellten Eigenschaften von GroEL-Substraten kann bei dieser Ausführungsform der erfindungsgemäßen Verfahren die GroELabhängige Faltung von einem in vitro exprimierten Protein durch Analyse seiner Sekundärstruktur, insbesondere der SCOP- und CATH-Domänen, sowie seines Molekulargewichts vorhergesagt werden.

Ein weiteres gut untersuchtes Chaperonsystem, bezüglich dessen die zu exprimierende Proteinsequenz analysiert werden kann, ist das DnaK/DnaJ/GRPL-Chaperon. DnaK gehört zur HSP70-Proteinfamilie. Das DnaK-System ist das Hauptchaperon, das die Aggregierung eines Großteils von thermolabilen Proteinen verhindert. Die Bindungsstellen und ein Konsensus-Erkennungsmotiv wurden durch Analyse von 37 biologisch relevanten prokaryontischen und eukaryontischen Proteinen abgeleitet, die natürliche Substrate von DnaK oder HSP70 sind (S. Rüdiger et al., EMBO J. 1997, 16:1501-1507). Die allgemeinen Merkmale der Substratbindenden Stellen von HSP70-Proteinen sind in der HSP70-Familie und somit auch in DnaK konserviert. Das durch DnaK erkannte Konsensusmotiv besteht aus einem zentralen hydrophoben Kern von vier bis fünf Resten, insbesondere Leucin, Isoleucin, Valin, Phenylalanin und Tyrosin, zusammen mit zwei flankierenden Regionen, die reich an basischen Resten sind.

Proteine des sekretorischen Stoffwechselwegs, die in das Periplasma oder das endoplasmatische Retikulum transportiert werden, werden durch Signalsequenzen markiert. Schätzungsweise etwa 10% aller Proteine in menschlichen und Arabidopsis-Zellen sind sekretorische Proteine. Die Signalsequenzen befinden sich üblicherweise am N-Terminus, haben eine Länge von etwa 20 - 30 Aminosäuren und sind in Prokaryonten und Eukaryonten gut konserviert. Diese Sequenzen haben eine gemeinsame Struktur, die aus einer positiv geladenen n-Region, gefolgt von einer hydrophoben h-Region und einer neutralen, aber polaren c-Region bestehen. Das Signalpeptid wird durch eine spezifische Protease gespalten, während das Polypeptid durch die Membran bewegt wird. Sekretorische Proteine enthalten häufig DisulfidBindungen, die nicht in der reduzierenden Umgebung eines zellfreien Lysats gebildet werden können. Somit ist die Löslichkeit und Funktionalität dieser Proteine bei ihrer Expression in einem zellfreien Translationssystem stark vermindert.

Somit wird bei einer weiteren bevorzugten Ausführungsform des erfindungsgemäßen Verfahrens die translatierte Aminosäuresequenz hinsichtlich Signalsequenzen und den entsprechenden Spaltstellen analysiert. Dabei wird vorzugsweise der SigP-Algorithmus (H. Nielsen et al., Protein Eng. 1997, 10: 1-6) verwendet. Die Vorhersage von Signalpeptid-Spaltstellen ist für Gram-negative bakterielle Sequenzen sehr präzise, während die Vorhersagen für eukaryontische und Grampositive bakterielle Sequenzen im Allgemeinen weniger genau sind, da deren Spaltstellenmuster nicht derart konserviert sind. Basierend auf dieser Vorhersage können Informationen bereitgestellt werden, ob die Sequenz des zu exprimierenden Proteins bereits ein Signalpeptid enthält, das von dem reifen Produkt an der spezifizierten Position abgespalten werden sollte. Ferner können experimentelle Bedingungen vorgeschlagen werden, mit denen das Problem der Verhinderung der Disulfidbrücken-Bildung umgangen werden kann.

Das erfindungsgemäße Verfahren verbessert die Expressionseffizienz von prokaryontischen Expressionssystemen, beispielsweise von solchen, die den Translationsapparat von *E. coli* benutzen. Die Genauigkeit der Vorhersage liegt bei etwa 75%, kann jedoch in Einzelfällen niedriger oder höher liegen. Das erfindungsgemäße Verfahren kann herangezogen werden, um die Expression von Proteinen für wissenschaftliche und/oder kommerzielle Zwecke zu optimieren, zu beschleunigen und/oder zu verbilligen.

Selbstverständlich kann das erfindungsgemäßen Verfahren auch zur Vorhersage der Expressionseffizienz in eukaryontischen Expressionssystemen eingesetzt werden, wobei gegebenenfalls spezielle die Expressionseffizienz in eukaryontischen Systemen beeinflussende Attributwerte der Expressionskonstrukte zu berücksichtigen sind.

Das im Folgenden beschriebene Beispiel dient zur Veranschaulichung der Erfindung, darf aber keineswegs beschränkend ausgelegt werden.

### Beispiel:

Im Folgenden wird die Vorgehensweise zur Erstellung einer Vorhersage-Verknüpfung beschrieben, mit dem die Expressionseffizienz für Expressionskonstrukte basierend auf der kodierenden Sequenz berechnet werden kann.

### 1. Datenbasis

### 1.1 Sequenzgenerierung und -auswahl

Für die Durchführung von Expressionsexperimenten wurden Gensequenzen aus verschiedenen Organismen ausgewählt. Das Ziel bestand darin, einen repräsentativen Satz von prokaryontischen und eukaryontischen Genen bereitzustellen. Dazu wurden etwa 200 menschliche, 200 *E*. *coli-,* 100 pflanzliche, 100 virale und 100 Genfragmente aus *S*. *cerevisiae* einschließlich der 39 Basen beginnend mit dem zweiten Kodon bereitgestellt.

### 1.1.1 Sequenzquellen

Zur Extraktion der offenen Leseraster wurden die Pedant^{™}-Datenbanken von *A. thaliana, E. coli* und *S.cerevisiae* verwendet, wobei offene Leseraster mit den Begriffen "hypothetisch", "möglich", "fraglich", "schwache Ähnlichkeit", "Fragment", "Plasmid", "Patent" und "vorhergesagt" im Beschreibungstext nicht betrachtet wurden. Somit wurden 1341 *A. thaliana-,* 1605 *E. coli-* und 3909 *S.cerevisae-*Sequenzen erhalten. Die menschlichen und viralen Sequenzen wurden von der EMBL-Datenbank unter Verwendung des folgenden Queries erhalten.

```
 {EMBL} : [([Organism EQ text:homo;]) & ([Organism EQ
 text:sapiens;]) & (![AllText EQ text:hypothetical;]) &
 (![AllText EQ text:putative;])
 & ([AllText EQ text:complete;]) & (![AllText EQ
 text:chromosome;]) & (![AllText EQ text:arm;])
 &(![AllText EQ text:patent;])
 & (![AllText EQ text:fragment;]) & (![AllText EQ
 text:putative;]) & (![AllText EQ text:cosmid;]) &
 (![AllText EQ text:"like";])
 & ([FtKey EQ text:"cds";]) & (![AllText EQ text:"weak";])
 &(![AllText EQ text:"questionable";]) & (![AllText EQ
 text:"partial";])]
 bzw.
 ({EMBL} : [([Organism EQ text:virus;]) & (![AllText EQ
 text:hypothetical;]) & (![AllText EQ text:putative;]) &
 ([AllText EQ text:complete;])
 & (![AllText EQ text:chromosome;]) & (![AllText EQ
 text:arm;]) & (![AllText EQ text:patent ;]) & (![AllText EQ
 text:fragment;])
 & (![AllText EQ text:putative;]) & (![AllText EQ
 text:cosmid;]) & (![AllText EQ text:"like";]) & ([FtKey
 EQ text:"cds";])
 & (![AllText EQ text:"weak";]) &(![AllText EQ
 text: "questionable";]) & (![AllText EQ text: "partial";])]
 )
```

Auf diese Weise wurden 9.162 menschliche und 13.657 virale Sequenzen erhalten.

### 1.1.2 Selektionsverfahren

Für jeden Organismus wurden spezifische Gen-Subsequenzen extrahiert, die 39 Basen beginnend mit dem zweiten Kodon umfassten. Für jeden Organismus wurden die Subsequenzen unter Verwendung einer hierarchischen Clusterung klassifiziert. In Abhängigkeit von der Anzahl der Mitglieder jeder Klasse und der Gesamtzahl an gewünschten Subsequenzen wurden solche Sequenzen extrahiert, die sich nahe dem Klassendurchschnitt befanden.

Durch diese Vorgehensweise wurden 221 menschliche, 202 *E. coli-,* 116 *A. thaliana-,* 108 virale und 109 *S. cerevisiae-*Sequenzen erhalten. An das 3'-Ende aller Sequenzen wurde die Gensequenz des grünen Fluoreszenzproteins (Green Fluorescence Proteine, GFP) und ein Hexa-His-Tag angefügt. Über eine 2-stufige PCR-Strategie wurden sämtliche linearen Expressionskonstrukte hergestellt. Die 39 Basenpaare langen Sequenzen aus den fünf verschiedenen Organismen wurden dabei über die Primer der ersten PCR eingeführt. Aus den so hergestellten Konstrukten wurden die entsprechenden mRNA-Sequenzen als Datenbasis für die hier beschriebene Analyse abgeleitet. Alle Konstrukte wurden in einem zellfreien *E. coli*-Expressionssystem (RTS Rapid Translation System RTS 100 E. coli HY Kit,Roche Diagnostics) exprimiert.

### 1.2 Initiationsregion

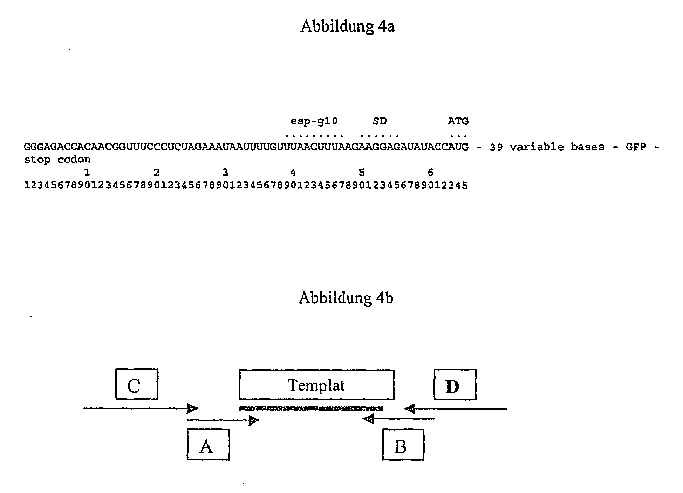

In Abbildung 4a ist die Initiationsregion aller Sequenzen gezeigt, Abb. 4b zeigt die PCR-Strategie.

### 1.3 Überblick über die Expressionswerte

Die Expressionsexperimente wurden mit dem RTS 100 E. coli HY Kit Expressionssystem von Roche Diagnostics GmbH durchgeführt. Die Expression von GFP wurde als interne Kontrolle gemessen. Alle Aktivitätsdaten wurden durch Proteindaten (SDS-PAGE/Coomasie Färbung) und/oder Western Blot Analysen verifiziert und abgeglichen. Im Folgenden sind alle Expressionsmengen als relativer prozentualer Anteil der Expression von GFP angegeben.

Drei Detektionstechniken wurden angewendet: Fluoreszenzdetektion des Fusionsproteins GFP, Densitometrie von Coomassie-gefärbten denaturierten Proteingelen und Western-Blot unter Verwendung eines Antikörpers gegen das C-terminale His-Tag. Der Coomassie-Wert wurde dann verwendet, wenn keine Fluoreszenz detektiert wurde, aber ein Westem-Blot-Signal vorlag. In den anderen Fällen wurde der Expressionswert aus dem Fluoreszenzsignal bestimmt.

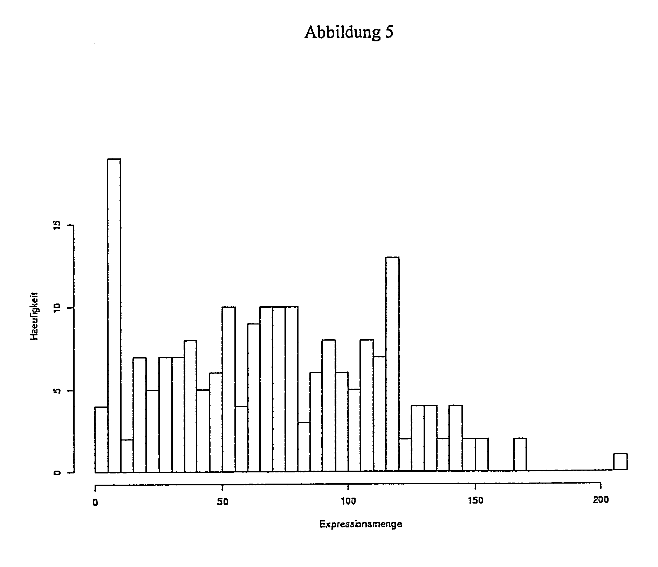

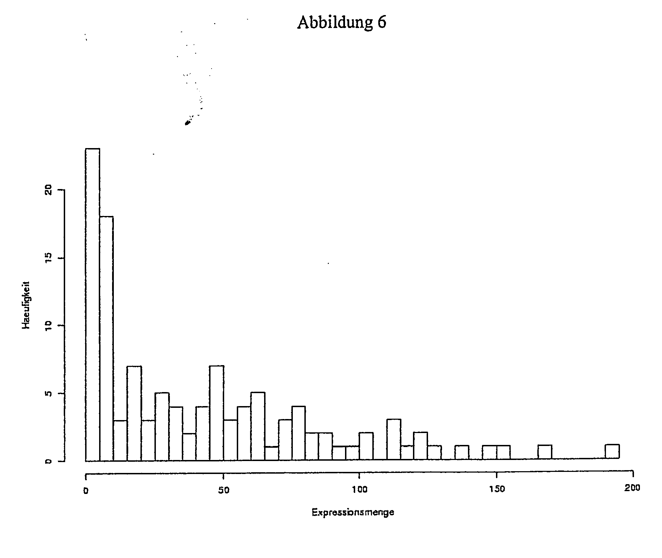

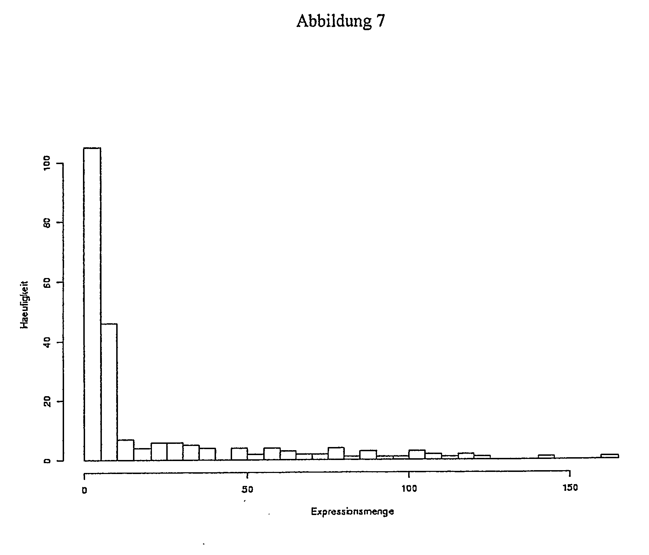

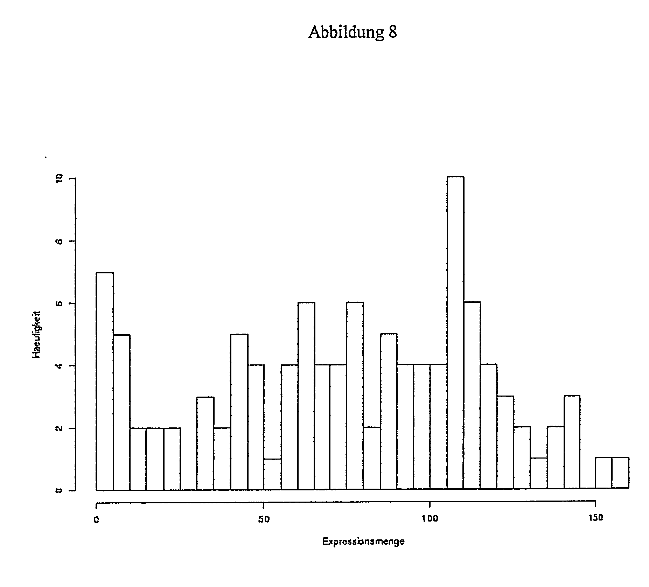

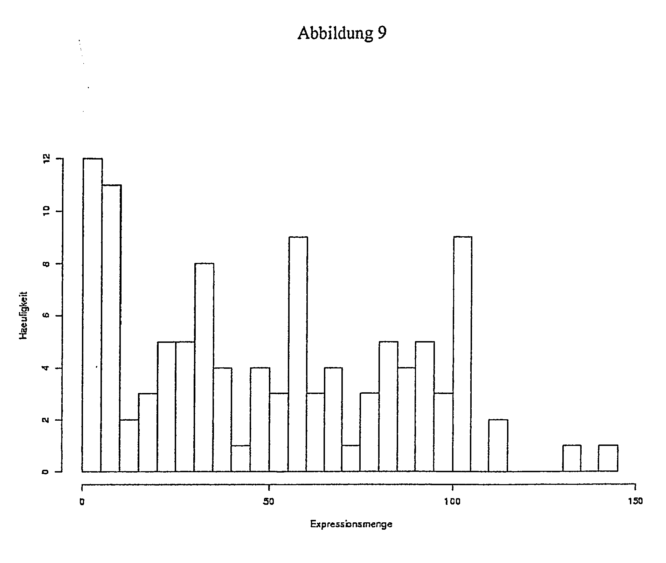

742 Sequenzen wurden in die Analyse mit einbezogen (siehe Tabelle 3 und Abbildung 2). Die relativen Expressionsmengen wurden in so genannte Expressionskategorien aufgeteilt: hoch (exp > 80), gut (30 < exp ≤80), niedrig (0 < exp ≤30) und keine (exp = 0). Die Abbildungen 5 bis 9 zeigen Expressionshistogramme für alle fünf Sequenzsätze.

**Tabelle 3: Expressionsdaten der 742 Sequenzen**

| Organismus | Mittlere Expression | hoch | gut | niedrig | keine | Gesamt |
|---|---|---|---|---|---|---|
| *Homo sapiens* | 20 | 16 | 29 | 79 | 95 | 219 |
| *E. coli* | 71 | 79 | 79 | 44 | 0 | 202 |
| *viral* | 51 | 30 | 40 | 32 | 6 | 108 |
| *A.thaliana* | 36 | 13 | 33 | 40 | 18 | 104 |
| *S.cerevisiae* | 75 | 52 | 39 | 14 | 4 | 109 |
| Gesamt | | 190 | 220 | 209 | 123 | 742 |

### 2. Sequenzattribute

Basierend auf der DNA-Sequenz wurden die folgenden Attribute bestimmt.

### 2.1 Primärstruktur

- Länge der Gensequenz
- Länge der mRNA-Sequenz
- GC-Gehalt:
   Der Gehalt an G oder C in der mRNA wird für verschiedene Sequenzabschnitte berechnet: gcX = GC-Gehalt für die Base X; gc_cont_X_Y = durchschnittlicher G/C-Gehalt zwischen den Basen X und Y (beispielsweise ist gc_cont_66_85 der Anteil an G oder C in den Basen 66 bis 85 der mRNA); gc_cont ist der Anteil an G oder C in der gesamten mRNA.
- Kodonadaptionsindex:
   Der Kodonadaptionsindex für *E. coli* gemäß Sharp und Li (1987) wird für verschiedene Sequenzabschnitte berechnet: caiX = der cai des Kodons X; cai_X_Y = der cai der Sequenz zwischen Kodon X und Y; cai = der cai der gesamten Gensequenz.
- Signal P-Werte:
   Signal P ist ein Programm zur Identifizierung von Signalpeptiden von sekretorischen Proteinen (http://www.cbs.dtu.dk/services/SignalP). Signalpeptide haben eine durchschnittliche Länge von etwa 26 Aminosäuren. Zur genauen Detektion von Sequenzpeptiden durch Signal P-Werte ist es erforderlich, eine Aminosäuresequenz mit einer Länge von 50 bis 70 Resten einzugeben. Deswegen wurden die ersten 70 Aminosäuren der ursprünglichen Gensequenzen, aus denen die 39 Basen für die Expressionsexperimente genommen wurden, als Eingabedaten zur Ermittlung der Signal P-Werte bereitgestellt. Falls Signal P ein Signalpeptid detektiert hat, lagen nur die ersten 13 Aminosäuren tatsächlich in den Expressionsexperimenten vor. Als Ausgaben liefert Signal P unter anderem die Werte meanS_val und maxY_val, die das Vorliegen von Signalsequenzen anzeigen.
- Anzahl der transmembranen Helices des Translationsprodukts, bestimmt durch den ALOM2-Algorithmus.

Im Folgenden werden die Abkürzungen pI für isoelektrischer Punkt des Translationsprodukts, bX für Base an Position X der Gensequenz, coX für Kodon X der Gensequenz und aaX für Aminosäure X des translatierten Proteinprodukts verwendet.

### 2.2 Sekundärstruktur

Die Vorhersage der Sekundärstruktur der mRNA wurde mit der Software VIENNA RNA PACKAGE (Version 1.3, Ivo Hofacker, Institut für theoretische Chemie, Währingerstr. 17, 1090 Wien, Österreich) mit den voreingestellten Energieparametem und einer mRNA-Länge von 300 Basen durchgeführt. ppX ist die Bindungswahrscheinlichkeit der Base X; ppwX ist die Energie-gewichtete Bindungswahrscheinlichkeit der Base X unter Berücksichtigung der Stabilität der Schleife, in der die Base sich befindet; ppweX ist die Energie-gewichtete Bindungswahrscheinlichkeit der Base X multipliziert mit der Energie der Schleife, in der sie sich befindet.

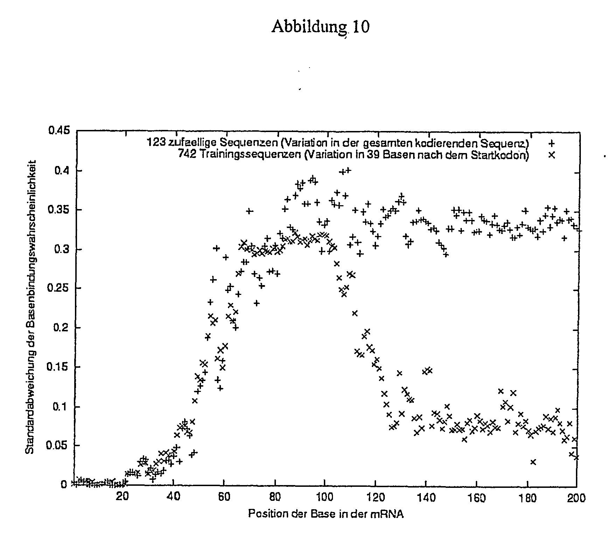

Die Standardabweichungen der Bindungswahrscheinlichkeiten ppX in dem Datensatz sind als Kreuze in Abbildung 10 gezeigt.

### 3. Identifizierung wichtiger Attribute

Ein bevorzugter Ansatz zum Auffinden quantitativer Attribute, die das gemessene Expressionslevel beeinflussen, ist die Berechnung der Korrelationswerte mit der Expressionsmenge. Zur Berechnung des Korrelationswertes wurden alle 742 Trainingdatensätze eingeschlossen. Im Allgemeinen befindet sich der Korrelationswert zwischen -1 und +1, wobei eine positive Korrelation bedeutet, dass das Expressionslevel umso höher ist, je höher der Attributwert ist, während eine negative Korrelation bedeutet, dass die Expressionsmenge umso niedriger ist, je höher der Attributwert ist.

### 3.1 Quantitative Primärstruktur-Attribute

Von allen Primärstrukturattributen weist der GC-Gehalt, insbesondere im Bereich zwischen den Basen 66 und 85 die deutlichste Korrelation mit der Expressionsmenge auf (siehe Tabelle 4).

**Tabelle 4: Primärstrukturattribute, die hohe Korrelationen mit der Expressionsmenge zeigen.**

| Attribut | Korrelation |
|---|---|
| gc_cont_41_80 | - 0,55 |
| gc_cont_81_120 | - 0,37 |
| gc_cont | - 0,51 |
| gc_cont_66_85 | - 0,56 |
| gc_cont_86_105 | - 0,33 |
| maxY_val | - 0,29 |
| meanS_val | - 0,32 |
| gc66 | - 0,31 |
| gc71 | - 0,26 |
| gc77 | - 0,27 |
| gc80 | - 0,26 |

Bei anderen berechneten quantitativen Primärstruktur-Attributwerten wie dem Kodonadaptionsindex ist die Korrelation z.T. weniger ausgeprägt, kann aber in Einzelfällen ebenfalls von Bedeutung sein.

### 3.2 Quantitative Sekundärstruktur-Attribute

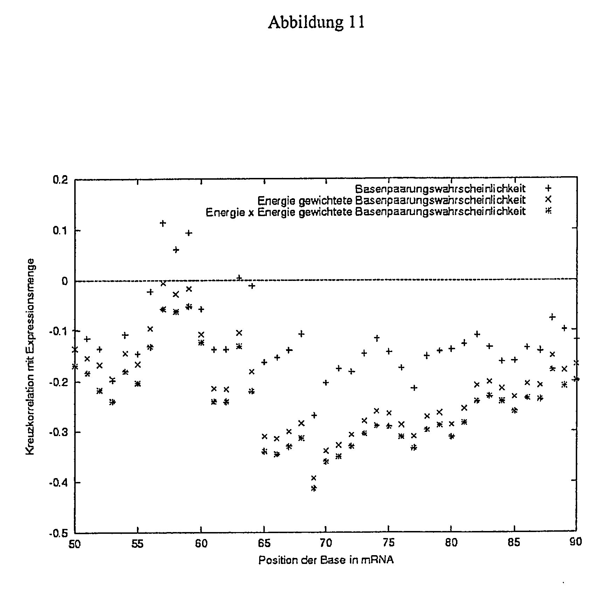

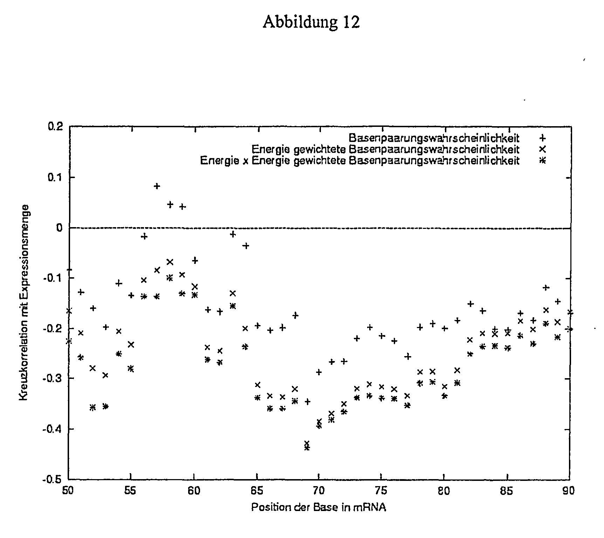

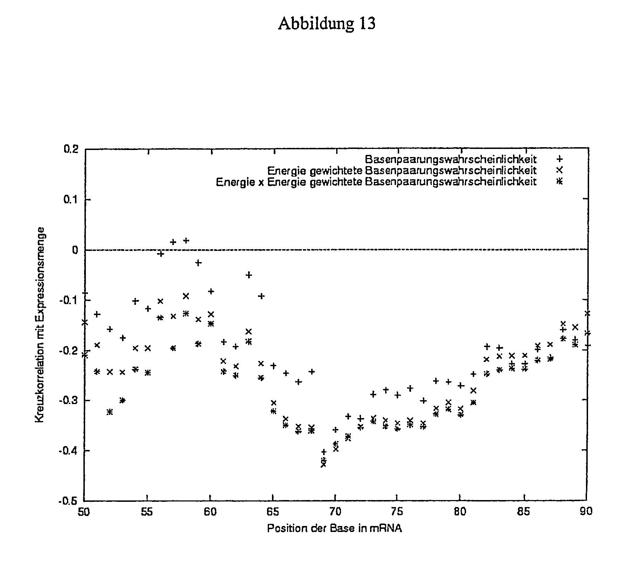

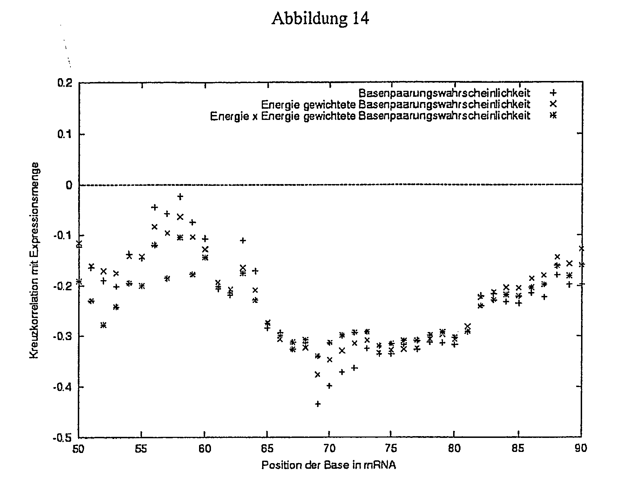

Hohe Korrelationswerte für die Basenpaarungswahrscheinlichkeiten von bestimmten Basen mit der Expressionsmenge sind in Tabelle 5 gezeigt. Die höchsten Werte liegen in einem Sequenzbereich mit einer Länge von etwa 20 Basen in unmittelbarer Nachbarschaft des Startkodons (siehe ebenso Abbildungen 11 bis 14).

**Tabelle 5: Korrelationswerte für Sekundärstrukturattribute bestimmt bei T = 60°C.**

| Base | pp | ppw |
|---|---|---|
| 65 | -0,23 | -0,30 |
| 66 | -0,25 | -0,34 |
| 67 | -0,26 | -0,35 |
| 68 | -0,24 | -0,35 |
| 69 | -0,40 | -0,43 |
| 70 | -0,36 | -0,40 |
| 71 | -0,33 | -0,38 |
| 72 | -0,34 | -0,35 |
| 73 | -0,29 | -0,34 |
| 74 | -0,28 | -0,34 |
| 75 | -0,29 | -0,35 |
| 76 | -0,28 | -0,34 |
| 77 | -0,30 | -0,35 |
| 78 | -0,26 | -0,32 |
| 79 | -0,26 | -0,30 |
| 80 | -0,27 | -0,32 |

Die Abbildungen 11 bis 14 zeigen die drei Arten von Sekundärstrukturattributen für die Basen 50 bis 90 (es gibt keine weiteren höheren Korrelationsbereiche in dem Basenbereich 1 bis 200). Vier verschiedene Temperaturen wurden bei der Sekundärstrukturvorhersage verwendet. Basenpositionen mit hohen Korrelationswerten sind für die Attribute pp, ppw und ppwe relativ beständig gegen Variationen in der Vorhersagetemperatur. Die Korrelationswerte für die drei Arten unterscheiden sich mehr für niedrige Temperaturen und konvergieren bei höheren Temperaturen. Die Energie-gewichteten Attribute ppw und ppwe zeigen im Allgemeinen höhere Korrelationswerte.

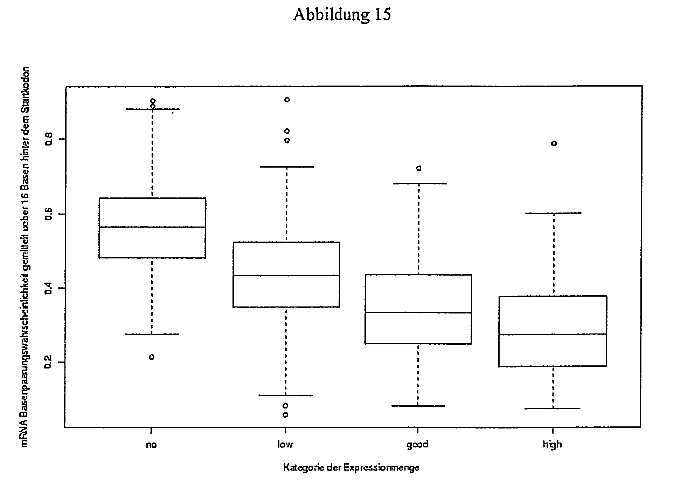

Die Paarungswahrscheinlichkeiten des Basenbereichs 65 bis 80 wurden gemittelt. Abbildung 15 zeigt den Box-Plot dieses Mittels ppav gegen die jeweiligen Expressionskategorien. Der Korrelationswert von ppav und der Expressionsmenge ist -0,537.

### 4. Regressionsmodelle

Basierend auf den vorstehend erhaltenen Trainingsdaten wurde mit Hilfe von Regressionsmodellen ein funktioneller Zusammenhang zwischen abhängigen und unabhängigen Variablen dargestellt. Als unabhängige Variablen wurden in diesem Beispiel die Werte der quantitativen Sequenzattribute verwendet, während die abhängige Variable der Expressionswert ist. Im Rahmen dieses Beispiels wurden nur lineare multi-variable Modelle berücksichtigt, das heißt die Koeffizienten wurden als linear festgelegt. Selbstverständlich sind jedoch auch nicht lineare Varianten denkbar. Zur Verbesserung des Fits wurde ein Polynom dritter Ordnung angewendet (siehe Abbildung 3).

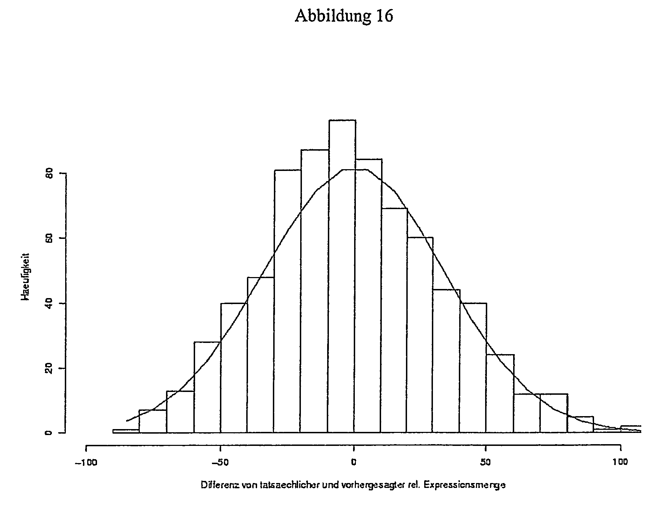

Die Histogramme der Abweichungen der vorhergesagten von den tatsächlichen Expressionswerten sind in Abbildung 16 gezeigt. Es ist eine Gauss-Kurve, die um den Nullpunkt mit einer Standardabweichung von etwa 0,33 REE zentriert ist. Etwa 68% (der Bereich unter der Gauss-Kurve zwischen ±0,33 REE) der Fälle liegen in einem Bereich ± 0,33 REE um den vorhergesagten Wert.

Die Genauigkeit, erhalten aus der Summe aller richtig vorhergesagten Fälle dividiert durch die Summe aller vorhergesagten Fälle, beträgt 0,79.

Um die Vorhersagegenauigkeit nochmals zu überprüfen, wurde der Fit unter Verwendung von nur 80% von zufällig ausgewählten Fällen der Trainingsdaten wiederholt. Die vorhergesagten Expressionswerte der verbleibenden 20% der Daten wurden dann mit den tatsächlichen Expressionswerten verglichen. Diese Analyse führte zu einer Breite der Gauss-Kurve von etwa 0,40 REE (Daten nicht gezeigt).

### 5. Entscheidungsbäume

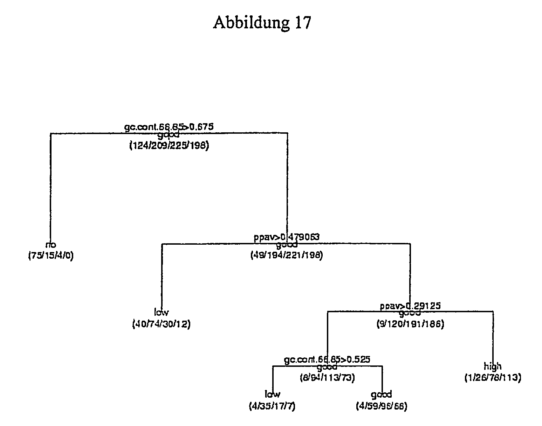

Ein alternativer Weg zur Vorhersage sind Computer-lernende Verfahren, die einen Entscheidungsbaum aus einem Satz von Fällen, die zu bekannten Klassen gehören, aufbauen (J.R. Quinlan, C4.5: Programs for Machine Learning, Morgan Kaufmann, 1993). An jedem Knotenpunkt in dem Baum wurde eine Klassifizierung basierend auf den Werten eines der Attribute getroffen. Um ein Blatt des Baums zu erreichen, muss eine Reihe von Entscheidungen getroffen werden. Die vier Kategorien an Expressionswerten bilden die Klassen "keine", "niedrig", "gut" und "hoch" wie vorstehend definiert. Die Abbildung 17 zeigen Entscheidungsbäume, die Tabelle 6 die entsprechenden Vorhersagegenauigkeiten. In erstere Annäherung ist es ausreichend, nur die Attribute ppav und gc_cont_66_85 einzuschließen, um den Entscheidungsbaum abzuleiten.

**Tabelle 6: Genauigkeit der Vorhersage durch den Entscheidungsbaum in Abbildung 17.**

| Expressionsklasse | Klassifikationswahrscheinlichkeit keine | Klassifikationswahrscheinlichkeit niedrig | Klassifikationswahrscheinlichkeit gut | Klassifikationswahrscheinlichkeit hoch |
|---|---|---|---|---|
| keine | 0.8 | 0.16 | 0.04 | 0 |
| niedrig | 0.16 | 0.52 | 0.23 | 0.09 |
| gut | 0.02 | 0.27 | 0.43 | 0.28 |
| hoch | 0.00 | 0.12 | 0.36 | 0.52 |

### 6. Diskussion

Mittels der in diesem Beispiel beschriebenen Experimente wird ein Datensatz bereitgestellt, der eine ausreichende Sequenzvariabilität in dem Sequenzbereich aufweist, der dem Translationsstartkodon benachbart ist, das heißt in den 39 Basen downstream des Translationsstarts. Der gesamte Rest der DNA ist für alle 742 Sequenzen konstant.

In dem Datensatz wurde ein breiter Bereich der Proteinexpressionsmenge von keiner Expression bis sehr hoher Expression beobachtet. Ausgehend von den vorgegebenen Sequenzen wurde ein Pool von mehreren 100 Attributwerten bestimmt. Attribute, die mit der Menge des Translationsprodukts korrelieren bzw. diese beeinflussen, wurden ausgewählt. Diese sind hauptsächlich der G/C-Gehalt und die Paarungswahrscheinlichkeit in der mRNA-Sekundärstruktur der ersten 20 Basen hinter dem Translationstartkodon

Ausgehend von diesem Untersatz an Attributen wurden Regressionsmodelle und Entscheidungsbäume erstellt. Bei einer gegebenen Sequenz, die aus derselben Expressionsvektorklasse wie in den verwendeten Trainingssequenzen stammt, kann eine Vorhersage der erwarteten Menge an Translationsprodukt in einem prokaryontischen System getroffen werden. Die Vorhersagegenauigkeit wird durch die Wahrscheinlichkeit beschrieben, dass die Expressionsmenge in einem gegebenen Bereich liegt. Die Wahrscheinlichkeit, dass die Expressionsmenge in einem Bereich von 40 Expressionseinheiten (100 entspricht der Expressionsmenge von GFP) von der vorhergesagten Expression liegt, beträgt zwei Drittel. Ausgehend von dieser Verteilung können andere Fragestellungen, die den Erfolg der Expression in prokaryontischen Translationssystemen betreffen, beantwortet werden.

Alternativ kann die Vorhersagegenauigkeit wie vorstehend beschrieben durch Bestimmung der Anzahl von richtig und falsch vorhergesagten Testfällen beschrieben werden. Abhängig vom Testdatensatz schwankt die Genauigkeit zwischen 65 und 85%.

### Abbildungen

Abb. 1a - 1c: Generierung von zirkulären und linearen Expressionskonstrukten.
Abb. 2: Histogramm der Expressionswerte von 742 Sequenzen, die lediglich in den 39 Basen ausgehend von dem zweiten Kodon variierten, angegeben als prozentualer Anteil der GFP-Expression.
Abb. 3: Dreidimensionaler Scatter-Plot der Expressionsmenge gegen den gemittelten G/C-Gehalt und die Basenpaarungswahrscheinlichkeit der mRNA-Sekundärstruktur.
Abb. 4: a) Initiationsregion aller im Beispiel gezeigten Sequenzen. esp-g10: Initiations-Enhancer-Region; SD: Shine-Dalgarno-Sequenz; ATG: TranslationsStartkodon; b) Primerdesign für die Expressions-PCR, mit den äußeren Primern 5'- = C, enthaltend T7 Promotor, Gen 10, RBS, und 3'- = D, enthaltend einen nicht translatierten Spacer und einen T7-Transkriptionsterminator; sowie den inneren Primern 5'- = A, enthaltend RBS, ATG und eine genspezifische Sequenz, und 3'- = B, enthaltend ein genspezifsche Sequenz and eine Spacerregion.
Abb. 5: Histogramm der Expressionswerte der Sequenzen aus *E. coli.*
Abb. 6: Histogramm der Expressionswerte der Sequenzen aus *A. thaliana.*
Abb. 7: Histogramm der Expressionswerte der humanen Sequenzen.
Abb. 8: Histogramm der Expressionswerte der Sequenzen aus *S*. *cerevisiae.*
Abb. 9: Histogramm der Expressionswerte der viralen Sequenzen.
Abb. 10: Darstellung der Standardabweichungen der Bindungswahrscheinlichkeiten ppX.
Abb. 11-14: Darstellung der Korrelationswerte für die Basenpaarungswahrscheinlichkeiten bei verschiedenen Temperaturen von bestimmten Basen mit der Expressionsmenge.
Abb. 15: Box-Plot des Mittels ppav gegen die Expressionskategorien "keine", "niedrig", "gut" und "hoch".
Abb. 16: Histogramme der Abweichungen der vorhergesagten von den tatsächlichen Expressionswerten.
Abb. 17: Darstellung eines Entscheidungsbaums zur Vorhersage der Expressionswahrscheinlichkeit.

## Patentansprüche

1. Verfahren zur Vorhersage der Expressionseffizienz bei der Herstellung eines Proteins in einem Expressionssystem, umfassend die folgenden Schritte:
A) Generierung von mindestens einem Expressionskonstrukt umfassend eine für das Protein kodierende Sequenz und flankierende Regulatorsequenzen;
B) Bestimmung von mindestens einem die Expressionseffizienz beeinflussenden Attributwert des generierten Expressionskonstrukts, wobei der Attributwert ausgewählt wird aus quantitativen Primärsüukturmerkmalen, qualitativen Primärstrukturmerkmalen und quantitativen Sekundärstrukturmerkmalen; und
C) Berechnung der Expressionseffizienz für das generierte Expressionskonstrukt, indem die Expressionseffizienz über einen Vorhersage-Algorithmus mit dem mindestens einen in Schritt B) bestimmten Attributwert verknüpft wird, wobei der Vorhersage-Algorithmus zur Berechnung der Expressionseffizienz aus der Abhängigkeit experimentell ermittelter Expressionsausbeuten von Expressionskonstrukten in dem Expressionssystem von den in Schritt B) bestimmten Attributwerten der Expressionskonstrukte durch multiple Regression, mittels eines neuronalen Netzes, mittels eines Bayesschen Netzes, und/oder mittels eines Computer-lernenden Verfahrens, das einen Entscheidungsbaum aus einem Satz von Fällen, die zu bekannten Klassen gehören aufbaut, abgeleitet worden ist.

2. Verfahren nach Anspruch 1,
**dadurch gekennzeichnet, dass** ein prokaryontisches Expressionssystem verwendet wird.

3. Verfahren nach Anspruch 2,
**dadurch gekennzeichnet, dass** als prokaryontisches Expressionssystem prokaryontische Zellen oder ein aus prokaryontischen Zellen gewonnener Extrakt verwendet werden.

4. Verfahren nach Anspruch 3,
**dadurch gekennzeichnet, dass** als prokaryontische Zelle oder für die Herstellung eines Zell-Extrakts E *coli* verwendet wird.

5. Verfahren nach einem der vorangehenden Ansprüche,
**dadurch gekennzeichnet, dass** ein Primärstruktur-Merkmal der G/C-Gehalt von Teilbereichen oder dem gesamten Bereich des Expressionskonstrukts ist.

6. Verfahren nach einem der vorangehenden Ansprüche,
**dadurch gekennzeichnet, dass** ein Primärstruktur-Merkmal die erste Base des zweiten Kodons der kodierenden Sequenz und/oder die Basenfolge des zweiten Kodons ist.

7. Verfahren nach einem der vorangehenden Ansprüche,
**dadurch gekennzeichnet, dass** ein Sekundärstruktur-Merkmal die Basenpaarungswahrscheinlichkeit für mindestens eine der Basen der mRNA-Sequenz ist.

8. Verfahren nach Anspruch 7,
**dadurch gekennzeichnet, dass** das Sekundärstruktur-Merkmal die Basenpaarungswahrscheinlichkeit für mindestens eine der Basen der mRNA-Sequenz im Bereich von 100 Basen vor und nach dem Startkodon ist.

9. Verfahren nach Anspruch 7 oder 8,
**dadurch gekennzeichnet, dass** das Sekundärstruktur-Merkmal die Basenpaarungswahrscheinlichkeit für mindestens eine der Basen der mRNA-Sequenz im Bereich von 60 Basen vor und nach dem Startkodon ist.

10. Verfahren nach einem der vorangehenden Ansprüche,
**dadurch gekennzeichnet, dass** die Expressionseffizienzen für Expressionskonstrukte bestimmt werden, die sich von der nativen, das herzustellende Protein kodierenden mRNA-Sequenz durch mindestens einen Basenaustausch unterscheiden.

11. Verfahren nach Anspruch 10,
**dadurch gekennzeichnet, dass** der oder die Basenaustausche in der für das Protein kodierenden mRNA-Sequenz zu identischen Aminosäuren oder konservativen Aminosäureaustauschen im Protein führen.

12. Verfahren nach Anspruch 10,
**dadurch gekennzeichnet, dass** der oder die Basenaustausche in der für das Protein kodierenden Sequenz der mRNA zur Substitution, Insertion oder Deletion durch eine oder mehrere der 20 natürlich vorkommenden Aminosäuren im Protein führen.

13. Verfahren nach einem der Ansprüche 10 bis 12,
**dadurch gekennzeichnet, dass** sich die Basenaustausche innerhalb der ersten 30 Kodons der translatierten Region der für das Protein kodierenden mRNA-Sequenz befinden.

14. Verfahren nach Anspruch 13,
**dadurch gekennzeichnet, dass** sich die Basenaustausche innerhalb der ersten 15 Kodons der translatierten Region der für das Protein kodierenden mRNA-Sequenz befinden.

15. Verfahren nach Anspruch 13,
**dadurch gekennzeichnet, dass** sich die Basenaustausche innerhalb der ersten 7 Kodons der translatierten Region der für das Protein kodierenden mRNA-Sequenz befinden.

16. Verfahren nach einem der Ansprüche 10 bis 15,
**dadurch gekennzeichnet, dass** die Expressionseffizienzen für Expressionskonstrukte bestimmt werden, die sich von der nativen, das herzustellende Protein kodierenden mRNA durch Deletion von Basen und/oder Insertion von Basen unterscheiden.

17. Verfahren nach einem der vorangehenden Ansprüche,
**dadurch gekennzeichnet, dass** die Generierung der Expressionskonstrukte unter Berücksichtigung der gewünschten Klonierungsstrategie; der Inkorporierung von Reinigungs- und/oder Detektions-Tags; und/oder der Anzahl bzw. Art der erlaubten Aminosäuresubstitutionen erfolgt.

18. Verfahren nach einem der vorangehenden Ansprüche,
**dadurch gekennzeichnet, dass** der G/C-Gehalt und/oder die Basenpaarungswahrscheinlichkeiten als unabhängige Variablen bei der multiplen Regression eingesetzt werden.

19. Verfahren nach einem der vorangehenden Ansprüche,
**dadurch gekennzeichnet, dass** die physiko-chemischen Eigenschaften der aus den Expressionskonstrukten abgeleiteten Translationsprodukte untersucht werden.

20. Verfahren nach Anspruch 19,
**dadurch gekennzeichnet, dass** die physiko-chemischen Eigenschaften ausgewählt werden aus der Gruppe bestehend aus Löslichkeit, Chaperonabhängigkeit und Produktfragmentierung durch Proteolyse, interne Fehlinitiation der Translation, vorzeitige Termination der Translation und/oder Auftreten von sekretorischen Signalsequenzen.

21. Verfahren nach einem der vorangehenden Ansprüche,
**dadurch gekennzeichnet, dass** die Expressionskonstrukte nach ungewünschten Fragmentierungsstellen analysiert werden und in Schritt A) Expressionskonstrukte generiert werden, mit denen die Fragmentierung minimiert wird.

22. Verfahren nach Anspruch 21,
**dadurch gekennzeichnet, dass** die ungewünschten Fragmentierungsstellen innerhalb der kodierenden Sequenz interne Initiationsstellen, vorzeitige Terminationsstellen und/oder seltene Kodon-Cluster sind.

23. Verfahren nach Anspruch 21,
**dadurch gekennzeichnet, dass** die ungewünschten Fragmentierungsstellen innerhalb des Proteinprodukts proteolytische Spaltstellen sind.

24. Verfahren nach einem der vorangehenden Ansprüche, umfassend die folgenden Schritte:
a) Bereitstellung einer das herzustellende Protein kodierenden Nukleinsäuresequenz;
b) Spezifizierung von Randbedingungen betreffend die Inkorporierung von Reinigungs- und/oder Detektions-Tags; und/oder die Anzahl bzw. Art von erlaubten Aminosäuresubstitutionen;
c) Generierung mindestens eines die für das Protein kodierende native Sequenz enthaltenden Expressionskonstrukts;
d) Generierung eines oder mehrerer veränderter Expressionskonstrukte durch Nukleotidsubstitutionen und/oder -insertionen und/oder -deletionen
e) Berechnung der Expressionseffizienz für jedes der Expressionskonstrukte durch wechselseitige Verknüpfung mit mindestens einem die Expressionseffizienz beeinflussenden Attributwert, ausgewählt aus quantitativen Primärstrukturmerkmalen, qualitativen Primärstrukturmerkmalen und quantitativen Sekundärstrukturmerkmalen;
f) Generierung von PCR-Primersequenzen;
g) Ausgabe der für das/die Expressionskonstrukte berechneten Expressionseffizienzen und/oder der PCR-Primersequenzen für die Expressionskonstrukte.

25. Verfahren nach einem der Ansprüche 1 bis 23, umfassend die folgenden Schritte:
a) Bereitstellung einer das herzustellende Protein kodierenden Nukleinsäuresequenz;
b) Spezifizierung von Randbedingungen betreffend die gewünschte Klonierungsstrategie, die Inkorporierung von Reinigungs- und/oder Detektions-Tags; und/oder die Anzahl bzw. Art von erlaubten Aminosäuresubstitutionen;
c) Auswahl eines geeigneten Expressionsvektors;
d) Generierung mindestens eines die für das Protein kodierende native Sequenz enthaltenden Expressionskonstrukts;
e) Generierung eines oder mehrerer veränderter Expressionskonstrukte durch Nukleotidsubstitutionen und/oder -insertionen und/oder -deletionen;
f) Berechnung der Expressionseffizienz für jedes der Expressionskonstrukte durch wechselseitige Verknüpfung mit mindestens einem die Expressionseffizienz beeinflussenden Attributwert, ausgewählt aus quantitativen Primärstrukturmerkmalen, qualitativen Primärstrukturmerkmalen und quantitativen Sekundärstrukturmerkmalen;
g) Ausgabe der für das/die Expressionskonstrukte berechneten Expressionseffizienzen.

26. Verfahren nach Anspruch 24 oder 25,
**dadurch gekennzeichnet, dass** Daten betreffend die physiko-chemischen Eigenschaften des Proteinprodukts und/oder Vorschläge zur ihrer Verbesserung bereitgestellt werden.

27. Verfahren nach einem der Ansprüche 24 bis 26,
**dadurch gekennzeichnet, dass** eine Anleitung der einzelnen Schritte zur Herstellung der Expressionskonstrukte bereitgestellt wird.

28. Verfahren nach einem der vorangehenden Ansprüche,
**dadurch gekennzeichnet, dass** mindestens ein Verfahrensschritt auf einem Computer durchgeführt wird.

29. Verfahren nach Anspruch 28,
**dadurch gekennzeichnet, dass** alle Verfahrensschritte auf einem Computer durchgeführt werden.

30. Computerlesbares Medium mit computerausführbaren Anweisungen zum Ausführen eines Verfahrens zur Vorhersage der Expressionseffizienz bei der Herstellung eines Proteins in Expressionssystemen, vorzugsweise prokaryontischen Expressionssystemen, wobei das Verfahren die folgenden Schritte umfasst:
A) Generierung von mindestens einem Expressionskonstrukt umfassend eine für das Protein kodierende Sequenz und flankierende Regulatorsequenzen;
B) Bestimmung von mindestens einem die Expressionseffizienz beeinflussenden Attributwert des generierten Expressionskonstrukts, wobei der Attributwert ausgewählt wird aus quantitativen Primärstrukturmerkmalen, qualitativen Primärstrukturmerkmalen und quantitativen Sekundärstrukturmerkmalen; und
C) Berechnung der Expressionseffizienz für das generierte Expressionskonstrukt, indem die Expressionseffizienz über einen Vorhersage-Algorithmus mit dem mindestens einen in Schritt B) bestimmten Attributwert verknüpft wird, wobei der Vorhersage-Algorithmus zur Berechnung der Expressionseffizienz aus der Abhängigkeit experimentell ermittelter Expressionsausbeuten von Expressionskonstrukten in dem Expressionssystem von den in Schritt B) bestimmten Attributwerten der Expressionskonstrukte durch multiple Regression, mittels eines neuronalen Netzes, mittels eines Bayesschen Netzes, und/oder mittels eines Computer-lernenden Verfahrens, das einen Entscheidungsbaum aus einem Satz von Fällen, die zu bekannten Klassen gehören aufbaut, abgeleitet worden ist.

31. Computerprogramm-Produkt, dergestalt ausgebildet, dass bei einer Ausführung des Computerprogramm-Produkts auf einem Computer ein Verfahren zur Vorhersage der Expressionseffizienz bei der Herstellung eines Proteins in Expressionssystemen, vorzugsweise prokaryontischen Expressionssystemen, ausgeführt wird, wobei das Verfahren die folgenden Schritte umfasst:
A) Generierung von mindestens einem Expressionskonstrukt umfassend eine für das Protein kodierende Sequenz und flankierende Regulatorsequenzen;
B) Bestimmung von mindestens einem die Expressionseffizienz beeinflussenden Attributwert des generierten Expressionskonstrukts, wobei der Attributwert ausgewählt wird aus quantitativen Primärstrukturmerkmalen, qualitativen Primärstrukturmerkmalen und quantitativen Sekundärstrukturmerkmalen; und
C) Berechnung der Expressionseffizienz für das generierte Expressionskonstrukt, indem die Expressionseffizienz über einen Vorhersage-Algorithmus mit dem mindestens einen in Schritt B) bestimmten Attributwert verknüpft wird, wobei der Vorhersage-Algorithmus zur Berechnung der Expressionseffizienz aus der Abhängigkeit experimentell ermittelter Expressionsausbeuten von Expressionskonstrukten in dem Expressionssystem von den in Schritt B) bestimmten Attributwerten der Expressionskonstrukte durch multiple Regression, mittels eines neuronalen Netzes, mittels eines Bayesschen Netzes, und/oder mittels eines Computer-lernenden Verfahrens, das einen Entscheidungsbaum aus einem Satz von Fällen, die zu bekannten Klassen gehören aufbaut, abgeleitet worden ist.

32. Verfahren zur Herstellung eines Proteins in Expressionssystemen, vorzugsweise in prokaryontischen Expressionssystemen, umfassend die folgenden Schritte:
a) Vorhersage der Expressionseffizienz für Expressionskonstrukte gemäß dem Verfahren nach einem der Ansprüche 1 bis 29;
b) Auswahl eines Expressionskonstrukts mit einer bestimmten, vorzugsweise der höchsten Expressionseffizienz;
c) Zelluläre oder zellfreie Expression des Proteins basierend auf dem Expressionskonstrukt aus Schritt b).

33. Verfahren nach Anspruch 32, ferner umfassend die Bereitstellung von Daten betreffend von physiko-chemischen Eigenschaften des Proteinprodukts.

## Claims

1. Method for predicting the expression efficiency in the preparation of a protein in an expression system, comprising the following steps:
A) generating at least one expression construct comprising a sequence coding for the protein and flanking regulatory sequences;
B) determining at least one attribute value of the generated expression construct influencing the expression efficiency, wherein the attribute value is selected from quantitative primary structure attributes, qualitative primary structure attributes and quantitative secondary structure attributes; and
C) calculating the expression efficiency of the generated expression construct by correlating the expression efficiency with the at least one attribute value determined in step B) using a prediction algorithm, wherein the prediction algorithm for calculating the expression efficiency is derived from the dependence of experimentally determined expression yields of expression constructs in the expression system on the attribute values of the expression constructs determined in step B) using multiple regression, a neural network, a Bayes network and/or a computer-learning method that constructs a decision tree out of a set of cases belonging to known classes.

2. Method according to claim 1,
**characterized in that** a prokaryotic expression system is used.

3. Method according to claim 2,
**characterized in that that** prokaryotic cells or an extract obtained from prokaryotic cells are used as prokaryotic expression system.

4. Method according to claim 3,
**characterized in that** *E. coli* is used as prokaryotic cell or for the preparation of a cell extract.

5. Method according to one of the preceding claims,
**characterized in that** a primary structure attribute is the G/C content in subregions or in the whole region of the expression construct.

6. Method according to one of the preceding claims,
**characterized in that** a primary structure attribute is the first base of the second codon of the coding sequence and/or the base sequence of the second codon.

7. Method according to one of the preceding claims,
**characterized in that** a secondary structure attribute is the base pairing probability for at least one of the bases in the mRNA sequence.

8. Method according to claim 7,
**characterized in that** the secondary structure attribute is the base pairing probability for at least one of the bases in the mRNA sequence in the region 100 bases upstream and 100 bases downstream of the start codon.

9. Method according to claim 7 or 8,
**characterized in that** the secondary structure attribute is the base pairing probability for at least one of the bases in the mRNA sequence in the region 60 bases downstream and 60 bases upstream of the start codon.

10. Method according to one of the preceding claims,
**characterized in that** the expression efficiencies are determined for expression constructs which differ from the native mRNA sequence coding for the protein to be prepared by at least one base substitution.

11. Method according to claim 10,
**characterized in that** the base substitution(s) in the mRNA sequence coding for the protein lead(s) to identical amino acids or conservative amino acid substitutions in the protein.

12. Method according to claim 10,
**characterized in that** the base substitution(s) in the mRNA sequence coding for the protein lead(s) to substitution, insertion or deletion by one or more of the 20 naturally occurring amino acids in the protein.

13. Method according to one of claims 10 to 12,
**characterized in that** the base substitutions occur within the first 30 codons of the translated region of the mRNA sequence coding for the protein.

14. Method according to claim 13,
**characterized in that** the base substitutions occur within the first 15 codons of the translated region of the mRNA sequence coding for the protein.

15. Method according to claim 13,
**characterized in that** the base substitutions occur within the first 7 codons of the translated region of the mRNA sequence coding for a protein.

16. Method according to one of claims 10 to 15,
**characterized in that** the expression efficiencies are determined for expression constructs which differ from the native mRNA coding for the protein to be prepared by deletion of bases and/or insertion of bases.

17. Method according to one of the preceding claims,
**characterized in that** the generation of expression constructs is performed upon consideration of the desired cloning strategy; the incorporation of purification and/or detection tags; and/or the number or type of permitted amino acid substitutions.

18. Method according to one of the preceding claims,
**characterized in that** the G/C content and/or the base pairing probability are used as independent variables in the multiple regression.

19. Method according to one of the preceding claims,
**characterized in that** the physico-chemical properties of the translation products derived from the expression constructs are analyzed.

20. Method according to claim 19,
**characterized in that** the physico-chemical properties are selected from the group consisting of solubility, chaperone dependency and product fragmentation by proteolysis, false internal initiation of translation, premature termination of translation and/or occurrence of secretory signal sequences.

21. Method according to one of the preceding claims,
**characterized in that** the expression constructs are analyzed for undesired fragmentation sites and expression constructs, with which the fragmentation is minimized, are generated in step A).

22. Method according to claim 21,
**characterized in that** the undesired fragmentation sites within the coding sequence are internal initiation sites, premature termination sites and/or rare codon clusters.

23. Method according to claim 21,
**characterized in that** the undesired fragmentation sites within the protein product are proteolytic cleavage sites.

24. Method according to one of the preceding claims, comprising the following steps:
a) providing a nucleic acid sequence which codes for the protein to be prepared;
b) specifying constraints for the incorporation of purification and/or detection tags; and/or the number or type of permitted amino acid substitutions;
c) generating at least one expression construct containing the native sequence coding for the protein;
d) generating one or more modified expression constructs by nucleotide substitutions and/or insertions and/or deletions;
e) calculating the expression efficiency for each of the expression constructs by mutual linkage with at least one of the attribute values influencing the expression efficiency, the attribute values being selected from quantitative primary structure attributes, qualitative primary structure attributes and quantitative secondary structure attributes;
f) generating PCR primer sequences;
g) outputting the expression efficiencies calculated for the expression constructs and/or the PCR primer sequences for the expression constructs.

25. Method according to one of claims 1 to 23, comprising the following steps:
a) providing a nucleic acid sequence which codes for the protein to be prepared;
b) specifying constraints for the desired cloning strategy, the incorporation of purification and/or detection tags; and/or the number or type of permitted amino acid substitutions;
c) selecting a suitable expression vector;
d) generating at least one expression construct containing the native sequence coding for the protein;
e) generating one or more modified expression constructs by nucleotide substitutions and/or insertions and/or deletions.
f) calculating the expression efficiency for each of the expression constructs by mutual linkage with at least one of the attribute values influencing the expression efficiency, the attribute values being selected from quantitative primary structure attributes, qualitative primary structure attributes and quantitative secondary structure attributes;
g) outputting the expression efficiencies calculated for the expression construct(s).

26. Method according to claim 24 or 25,
**characterized in that** data concerning the physico-chemical properties of the protein product and/or suggestions for their improvement are provided.

27. Method according to one of claims 24 to 26,
**characterized in that** an instruction for the single steps for preparing the expression constructs is provided.

28. Method according to one of the preceding claims,
**characterized in that** the at least one process step is performed on a computer.

29. Method according to claim 28,
**characterized in that** all process steps are performed on a computer.

30. Machine-readable medium with instructions which can be performed on a computer for the performance of a method for predicting the expression efficiency in the preparation of a protein in expression systems, preferably in prokaryotic expression systems, wherein the method comprises the following steps:
A) generating at least one expression construct comprising a sequence coding for the protein and flanking regulatory sequences;
B) determining at least one attribute value for the generated expression construct influencing the expression efficiency, wherein the attribute value is selected from quantitative primary structure attributes, qualitative primary structure attributes and quantitative secondary structure attributes; and
C) calculating the expression efficiency of the generated expression construct by correlating the expression efficiency with the at least one attribute value determined in step B) using a prediction algorithm, wherein the prediction algorithm for calculating the expression efficiency is derived from the dependence of experimentally determined expression yields of expression constructs in the expression system on the attribute values of the expression constructs determined in step B) using multiple regression, a neural network, a Bayes network and/or a computer-learning method that constructs a decision tree out of a set of cases belonging to known classes.

31. Computer program product designed such that a method for predicting the expression efficiency in the preparation of a protein in expression systems, preferably in prokaryotic expression systems, is performed when the computer program product is used on a computer, wherein the process comprises the following steps:
A) generating at least one expression construct comprising a sequence coding for the protein and flanking regulatory sequences;
B) determining at least one attribute value for the generated expression construct influencing the expression efficiency, wherein the attribute value is selected from quantitative primary structure attributes, qualitative primary structure attributes and quantitative secondary structure attributes; and
C) calculating the expression efficiency of the generated expression construct by correlating the expression efficiency with the at least one attribute value determined in step B) using a prediction algorithm, wherein the prediction algorithm for calculating the expression efficiency is derived from the dependence of experimentally determined expression yields of expression constructs in the expression system on the attribute values of the expression constructs determined in step B) using multiple regression, a neural network, a Bayes network and/or a computer-learning method that constructs a decision tree out of a set of cases belonging to known classes.

32. Method for the preparation of a protein in expression systems, preferably in prokaryotic expression systems, comprising the following steps:
a) predicting the expression efficiency of expression constructs according to the method of one of claims 1 to 29;
b) selecting an expression construct with a determined expression efficiency, preferably the highest expression efficiency;
c) cellular or cell-free expression of the protein based on the expression construct from step b).

33. Method according to claim 32, further comprising providing data concerning the physico-chemical properties of the protein product.

## Revendications

1. Procédé pour prévoir l'efficacité d'expression lors de la production d'une protéine dans un système d'expression comprenant les étapes suivantes :
A) Génération d'au moins une construction d'expression comprenant une séquence codant la protéine et les séquences régulatrices flanquantes ;
B) Détermination d'au moins une valeur d'attribut de la construction d'expression générée qui influence l'efficacité d'expression, ladite valeur d'attribut étant choisie parmi les caractéristiques structurales primaires quantitatives, les caractéristiques structurales primaires qualitatives et les caractéristiques structurales secondaires quantitatives ; et
C) Calcul de l'efficacité d'expression pour la construction d'expression générée, en reliant l'efficacité d'expression par un algorithme de prédiction à l'une au moins des valeurs d'attribut déterminées à l'étape B), l'algorithme de prédiction pour le calcul de l'efficacité d'expression étant dérivé de la dépendance des rendements d'expression des constructions d'expression dans le système d'expression calculés expérimentalement par rapport aux valeurs d'attribut des constructions d'expression déterminées dans l'étape B) par régression multiple, au moyen d'un réseau neuronal, au moyen d'un réseau bayésien et/ou au moyen d'un procédé d'apprentissage par ordinateur, qui construit un arbre de décision à partir d'une série de cas appartenant à des classes connues.

2. Procédé selon la revendication 1,
**caractérisé en ce qu'**un système d'expression procaryote est utilisé.

3. Procédé selon la revendication 2,
**caractérisé en ce que** des cellules procaryotes ou un extrait de cellules procaryotes sont utilisés en tant que système d'expression procaryote.

4. Procédé selon la revendication 3,
**caractérisé en ce que** *E. coli* est utilisé en tant que cellule procaryote ou pour la production d'un extrait cellulaire.

5. Procédé selon l'une quelconque des revendications précédentes,
**caractérisé en ce qu'**une caractéristique structurale primaire est la teneur en G/C de parties ou de la totalité de la construction d'expression.

6. Procédé selon l'une quelconque des revendications précédentes,
**caractérisé en ce qu'**une caractéristique structurale primaire est la première base du deuxième codon de la séquence codante et/ou la séquence des bases du deuxième codon.

7. Procédé selon l'une quelconque des revendications précédentes,
**caractérisé en ce qu'**une caractéristique structurale secondaire est la probabilité d'appariement des bases pour au moins une des bases de la séquence d'ARNm.

8. Procédé selon la revendication 7,
**caractérisé en ce que** la caractéristique structurale secondaire est la probabilité d'appariement des bases pour au moins une des bases de la séquence d'ARNm dans la région de 100 bases avant et après le codon d'initiation.

9. Procédé selon la revendication 7 ou 8,
**caractérisé en ce que** la caractéristique structurale secondaire est la probabilité d'appariement des bases pour au moins une des bases de la séquence d'ARNm dans la région de 60 bases avant et après le codon d'initiation.

10. Procédé selon l'une quelconque des revendications précédentes,
**caractérisé en ce que** les efficacités d'expression pour les constructions d'expression, qui se différencient de la séquence d'ARNm native codant la protéine à produire par au moins un échange de base, sont déterminées.

11. Procédé selon la revendication 10,
**caractérisé en ce que** le ou les échange(s) de base(s) dans la séquence d'ARNm codant la protéine aboutissent à des acides aminés identiques ou à des échanges conservateurs d'acides aminés dans la protéine.

12. Procédé selon la revendication 10,
**caractérisé en ce que** le ou les échange(s) de base(s) dans la séquence de l'ARNm codant la protéine aboutissent à une substitution, à une insertion ou à une délétion dans la protéine par un ou plusieurs des 20 acides aminés naturellement rencontrés.

13. Procédé selon l'une quelconque des revendications 10 à 12,
**caractérisé en ce que** les échanges de bases se situent dans les 30 premiers codons de la région traduite de la séquence d'ARNm codant la protéine.

14. Procédé selon la revendication 13,
**caractérisé en ce que** les échanges de bases se situent dans les 15 premiers codons de la région traduite de la séquence d'ARNm codant la protéine.

15. Procédé selon la revendication 13,
**caractérisé en ce que** les échanges de bases se situent dans les 7 premiers codons de la région traduite de la séquence d'ARNm codant la protéine.

16. Procédé selon l'une quelconque des revendications 10 à 15,
**caractérisé en ce que** les efficacités d'expression pour les constructions d'expression, qui se différencient de l'ARNm natif codant la protéine à produire par délétion de bases et/ou insertion de bases, sont déterminées.

17. Procédé selon l'une quelconque des revendications précédentes,
**caractérisé en ce que** la génération des constructions d'expression s'effectue considérant la stratégie de clonage désirée ; l'incorporation de tags de purification et/ou de détection; et/ou le nombre ou bien la nature des substitutions d'acides aminés autorisées.

18. Procédé selon l'une quelconque des revendications précédentes,
**caractérisé en ce que** la teneur en G/C et/ou les probabilités d'appariement des bases sont employées comme variables indépendantes lors de la régression multiple.

19. Procédé selon l'une quelconque des revendications précédentes,
**caractérisé en ce que** les propriétés physico-chimiques des produits de traduction dérivant des construction d'expression sont analysées.

20. Procédé selon la revendication 19,
**caractérisé en ce que** les propriétés physico-chimiques sont choisies à partir du groupe constitué par la solubilité, la dépendance aux protéines chaperones et la fragmentation de produit par protéolyse, l'initiation défaillante interne de la traduction, la terminaison précoce de la traduction et/ou l'apparition de séquences signal sécrétoires.

21. Procédé selon l'une quelconque des revendications précédentes,
**caractérisé en ce que** les constructions d'expression sont analysées relativement à des sites de fragmentation non désirés et des constructions d'expression avec lesquelles la fragmentation est minimisée sont générées à l'étape A).

22. Procédé selon la revendication 21,
**caractérisé en ce que** les sites de fragmentation non désirés dans la séquence codante sont des sites d'initiation internes, des sites de terminaison précoces et/ou des clusters de codons rares.

23. Procédé selon la revendication 21,
**caractérisé en ce que** les sites de fragmentation non désirés dans le produit protéique sont des sites de clivage protéolytiques.

24. Procédé selon l'une quelconque des revendications précédentes comprenant les étapes suivantes :
a) Préparation d'une séquence d'acide nucléique codant la protéine à produire ;
b) Spécification des conditions aux limites concernant l'incorporation de tags de purification et/ou de détection ; et/ou le nombre ou la nature des substitutions d'acides aminés autorisées ;
c) Génération d'au moins une construction d'expression contenant la séquence native codant la protéine ;
d) Génération d'une ou de plusieurs construction(s) d'expression modifiée(s) par substitutions et/ou insertions et/ou délétions de nucléotides ;
e) Calcul de l'efficacité d'expression pour chacune des constructions d'expression par liaison réciproque avec au moins une valeur d'attribut influençant l'efficacité d'expression, choisie parmi les caractéristiques structurales primaires quantitatives, les caractéristiques structurales primaires qualitatives et les caractéristiques structurales secondaires quantitatives ;
f) Génération de séquences d'amorces de PCR ;
g) Sortie des efficacités d'expression calculées pour la/les construction(s) d'expression et/ou des séquences d'amorces de PCR pour les constructions d'expression.

25. Procédé selon l'une quelconque des revendications 1 à 23 comprenant les étapes suivantes :
a) Préparation d'une séquence d'acide nucléique codant la protéine à produire;
b) Spécification des conditions aux limites concernant la stratégie de clonage désirée, l'incorporation de tags de purification et/ou de détection ; et/ou le nombre ou la nature des substitutions d'acides aminés autorisées ;
c) Choix d'un vecteur d'expression approprié ;
d) Génération d'au moins une construction d'expression contenant la séquence native codant la protéine ;
e) Génération d'une ou de plusieurs construction(s) d'expression modifiée(s) par substitutions et/ou insertions et/ou délétions de nucléotides ;
f) Calcul de l'efficacité d'expression pour chacune des constructions d'expression par liaison réciproque avec au moins une valeur d'attribut influençant l'efficacité d'expression, choisie parmi les caractéristiques structurales primaires quantitatives, les caractéristiques structurales primaires qualitatives et les caractéristiques structurales secondaires quantitatives ;
g) Sortie des efficacités d'expression calculées pour la/les construction(s) d'expression.

26. Procédé selon la revendication 24 ou 25,
**caractérisé en ce que** des données concernant les propriétés physico-chimiques du produit protéique et/ou des propositions pour leur amélioration sont fournies.

27. Procédé selon l'une quelconque des revendications 24 à 26,
**caractérisé en ce que** des directives concernant les différentes étapes conduisant à l'élaboration des constructions d'expression sont fournies.

28. Procédé selon l'une quelconque des revendications précédentes,
**caractérisé en ce que** au moins une étape du procédé est effectuée par ordinateur.

29. Procédé selon la revendication 28,
**caractérisé en ce que** toutes les étapes du procédé sont effectuées par ordinateur.

30. Média lisible par ordinateur avec des directives réalisables par ordinateur pour exécuter un procédé pour prévoir l'efficacité d'expression lors de la production d'une protéine dans des systèmes d'expression, de préférence dans des systèmes d'expression procaryotes, le procédé comprenant les étapes suivantes :
A) Génération d'au moins une construction d'expression comprenant une séquence codant la protéine et les séquences régulatrices flanquantes ;
B) Détermination d'au moins une valeur d'attribut de la construction d'expression générée qui influence l'efficacité d'expression, ladite valeur d'attribut étant choisie parmi les caractéristiques structurales primaires quantitatives, les caractéristiques structurales primaires qualitatives et les caractéristiques structurales secondaires quantitatives ; et
C) Calcul de l'efficacité d'expression pour la construction d'expression générée, en reliant l'efficacité d'expression par un algorithme de prédiction à l'une au moins des valeurs d'attribut déterminée à l'étape B), l'algorithme de prédiction pour le calcul de l'efficacité d'expression étant dérivé de la dépendance des rendements d'expression des constructions d'expression dans le système d'expression calculés expérimentalement par rapport aux valeurs d'attribut des constructions d'expression déterminées dans l'étape B) par régression multiple, au moyen d'un réseau neuronal, au moyen d'un réseau bayésien et/ou au moyen d'un procédé d'apprentissage par ordinateur, qui construit un arbre de décision à partir d'une série de cas appartenant à des classes connues.

31. Produit logiciel développé de telle manière que lors d'une mise en oeuvre du produit logiciel sur un ordinateur, un procédé pour prévoir l'efficacité d'expression lors de la production d'une protéine dans des systèmes d'expression, de préférence dans des systèmes d'expression procaryotes, est exécuté, le procédé comprenant les étapes suivantes :
A) Génération d'au moins une construction d'expression comprenant une séquence codant la protéine et les séquences régulatrices flanquantes ;
B) Détermination d'au moins une valeur d'attribut de la construction d'expression générée qui influence l'efficacité d'expression, ladite valeur d'attribut étant choisie parmi les caractéristiques structurales primaires quantitatives, les caractéristiques structurales primaires qualitatives et les caractéristiques structurales secondaires quantitatives ; et
C) Calcul de l'efficacité d'expression pour la construction d'expression générée, en reliant l'efficacité d'expression par un algorithme de prédiction à l'une au moins des valeurs d'attribut déterminées à l'étape B), l'algorithme de prédiction pour le calcul de l'efficacité d'expression étant dérivé de la dépendance des rendements d'expression des constructions d'expression dans le système d'expression calculés expérimentalement par rapport aux valeurs d'attribut des constructions d'expression déterminées dans l'étape B) par régression multiple, au moyen d'un réseau neuronal, au moyen d'un réseau bayésien et/ou au moyen d'un procédé d'apprentissage par ordinateur, qui construit un arbre de décision à partir d'une série de cas appartenant à des classes connues.

32. Procédé pour produire une protéine dans des systèmes d'expression, de préférence dans des systèmes d'expression procaryotes, comprenant les étapes suivantes :
a) Prévision de l'efficacité d'expression pour les constructions d'expression conformément au procédé selon l'une quelconque des revendications 1 à 29 ;
b) Sélection d'une construction d'expression ayant une efficacité d'expression définie, de préférence la plus élevée ;
c) Expression cellulaire ou exempte de cellules de la protéine basée sur la construction d'expression de l'étape b).

33. Procédé selon la revendication 32 comprenant de plus la mise à disposition de données concernant les propriétés physico-chimiques du produit protéique.
